(19) Europäisches Patentamt
European Patent Office
Office européen des brevets

(11) EP 4 582 130 A1

(12) **EUROPEAN PATENT APPLICATION**
published in accordance with Art. 153(4) EPC

(43) Date of publication:
**09.07.2025 Bulletin 2025/28**

(21) Application number: **23872340.7**

(22) Date of filing: **26.09.2023**

(51) International Patent Classification (IPC):
***A61M 25/10*** *(2013.01)*

(52) Cooperative Patent Classification (CPC):
**A61M 25/10**

(86) International application number:
**PCT/JP2023/034940**

(87) International publication number:
**WO 2024/071117 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.09.2022 JP 2022156478**

(71) Applicant: **Terumo Kabushiki Kaisha**
**Tokyo 151-0072 (JP)**

(72) Inventors:
- **MOTOSE, Yuji**
  **Fujinomiya-shi**
  **Shizuoka 418-0015 (JP)**
- **KOINUMA, Naoki**
  **Fujinomiya-shi**
  **Shizuoka 418-0015 (JP)**
- **SUGIKI, Tsutomu**
  **Fujinomiya-shi**
  **Shizuoka 418-0015 (JP)**

(74) Representative: **Casalonga**
**Casalonga & Partners**
**Bayerstraße 71/73**
**80335 München (DE)**

# (54) BALLOON CATHETER

(57) Provided is a highly flexible balloon catheter. The balloon catheter including a shaft that extends in an axial direction, and a balloon that is disposed on an outer periphery of the shaft, in which in a distal portion of the balloon, a crystallinity on a distal side is lower than a crystallinity on a proximal side.

*FIG. 3A*

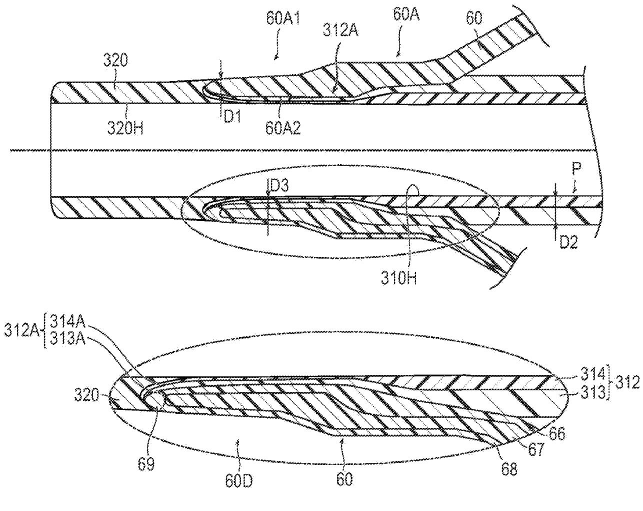




EP 4 582 130 A1

## Description

### Technical Field

The present invention relates to a balloon catheter.

### Background Art

In order to eliminate and treat constriction of a stenosis in a living body, a balloon catheter including a balloon that is introduced into a stenosis developed in a tubular cavity or lumen in a living body to expand the stenosis outward from the inside is used. For example, a balloon catheter is employed in percutaneous coronary intervention (PCI) in which a balloon is used to expand a stenosis of a coronary artery to improve blood flow.

Furthermore, a guiding catheter is first introduced and placed at a predetermined site in a blood vessel, and a treatment device such as a balloon catheter is inserted therein to perform a predetermined treatment.

A catheter has an elongated shaft member extending in an axial direction. Since the shaft member is introduced to a predetermined position such as the vicinity of a stenosis in a living body by a user (operator), it is necessary to have flexibility on the distal side so that the stress applied to the living body can be reduced while the hardness is sufficiently maintained in order to prevent buckling in the living body or to transmit an operation of the user to the distal end.

Therefore, for example, Patent Literature 1 discloses a configuration in which a soft distal tip is attached and fixed to a distal portion of an inner tube of a balloon catheter to provide flexibility at the distal portion. Furthermore, Patent Literature 2 discloses a configuration of a balloon catheter in which a balloon part is bonded across both the outer periphery of a soft distal tip and the outer periphery of a shaft part, and a distal end of the balloon part is formed in a tapered shape that tapers to a point.

### Citation List

### Patent Literatures

Patent Literature 1: JP H05-192410 A
Patent Literature 2: JP 2005-160536 A

### Summary of Invention

As described above, since the balloon catheter is required to have good passability in a living body, sufficient flexibility is necessary.

Therefore, an object of the present invention is to provide a balloon catheter with high flexibility.

Preferable aspects of the present invention are as follows.

(1) The balloon catheter includes a shaft that extends in an axial direction, and a balloon that is disposed on an outer periphery of the shaft, in which in a distal portion of the balloon, a crystallinity on a distal side is lower than a crystallinity on a proximal side.

(2) In the balloon catheter according to (1), the crystallinity of the distal side of the distal portion of the balloon preferably gradually decreases from the proximal side toward the distal side.

(3) In the balloon catheter according to (1) or (2), the crystallinity on the distal side of the distal portion of the balloon is preferably 1.5% to 6% lower than the crystallinity on the proximal side.

(4) In the balloon catheter according to any one of (1) to (3), in a cross-sectional view in the axial direction, the distal portion of the balloon preferably includes a wedge portion that is wedge-shaped, and the wedge portion preferably includes a parallel portion parallel to the axial direction.

(5) In the balloon catheter according to (4), the balloon preferably includes an inner layer, a base layer, and an outer layer, and in the parallel portion, a crystallinity of the outer layer is preferably lower than a crystallinity of the inner layer.

(6) In the balloon catheter according to (5), the inner layer and the outer layer preferably contain an elastomer.

(7) In the balloon catheter according to any one of (1) to (6), the balloon catheter further includes a distal member that is fused to a distal side of the shaft and is more flexible than the shaft, and a crystallinity of each of a proximal portion of the distal member and a distal region of the distal portion of the balloon is preferably less than 40%.

(8) In the balloon catheter according to any one of (1) to (7), a thickness at a distal end of the balloon positioned at a portion fused to the shaft is preferably equal to or smaller than a thickness of the shaft at a site closer to the proximal

side than a portion where the balloon is bonded to the shaft, and is preferably larger than a thickness of the shaft at a portion bonded to the balloon.

According to the above-described aspects of the present invention, it is possible to provide the balloon catheter with high flexibility.

Brief Description of Drawings

Fig. 1 is a diagram illustrating an overall configuration of a balloon catheter according to an embodiment of the present invention.
Fig. 2 is a diagram illustrating a cross section along an axial direction in the vicinity of a balloon of the balloon catheter according to the embodiment of the present invention.
Fig. 3A is a partially enlarged diagram of part A in Fig. 2.
Fig. 3B is a diagram of a balloon catheter according to a modification, which corresponds to Fig. 3A.
Fig. 4 is a diagram for explaining a method of manufacturing the balloon catheter according to the embodiment of the present invention.
Fig. 5 is a diagram of a balloon catheter according to an embodiment of the present invention, which corresponds to Fig. 3B.
Fig. 6 is a diagram of a balloon catheter according to an embodiment of the present invention, which corresponds to Fig. 3B.
Fig. 7 is a graph illustrating an IR spectrum of a resin 1 used for a balloon catheter of Example.
Fig. 8 is a graph illustrating an IR spectrum of a resin 2 used for the balloon catheter of Example.
Fig. 9 is a graph illustrating an IR spectrum of a resin 3 used for the balloon catheter of Example.
Fig. 10 is a graph illustrating an IR spectrum of a resin 4 used for the balloon catheter of Example.
Fig. 11 is a graph illustrating an IR spectrum of a resin 5 used for the balloon catheter of Example.
Fig. 12 illustrates images of measurement results of crystallinity distribution confirmed by imaging IR for balloon catheters of Example and Comparative Example.
Fig. 13 illustrates images of relationships between measurement results of crystallinity distribution confirmed by imaging IR for the balloon catheters of Example and Comparative Example, and individual members.
Fig. 14 illustrates images of appearance photographs and relationships between IR images and measurement positions in the balloon catheters of Example and Comparative Example.
Fig. 15 is a diagram illustrating results of a cantilever bending test for the balloon catheters of Example and Comparative Example.

Description of Embodiments

Hereinafter, embodiments of the present invention will be described with reference to the accompanying drawings. The present invention is not limited only to the following embodiments. In the description of the drawings, the same elements are denoted by the same reference numerals, and redundant description will be omitted. Furthermore, dimensional ratios in the drawings are exaggerated for convenience of description, and may be different from actual ratios.
In the present specification, a range from "X to Y" includes X and Y, and indicates "X or more and Y or less". Furthermore, a form in which two or three or more of preferable individual forms of the present invention described below are combined is also regarded as a preferable form of the present invention and disclosed in the present specification (that is, it is a legitimate basis for amendments).
In a case where the configurations and the relationships between the configurations in the cross-sectional diagrams are described, the descriptions will be of the cross-sectional view in an axial direction unless otherwise specified.
An aspect of the present invention is a balloon catheter including a shaft that extends in an axial direction, and a balloon that is disposed on an outer periphery of the shaft, in which in a distal portion of the balloon, a crystallinity on a distal side is lower than a crystallinity on a proximal side. According to the balloon catheter according to the aspect of the present invention, since the crystallinity on the distal side of the distal portion of the balloon is lower than the crystallinity on the proximal side, the balloon catheter is flexible and can improve the passability in a living body.
In the present specification, the balloon distal portion means a portion where the balloon and the shaft are bonded to each other. The distal side of the balloon distal portion means a region closer to the distal side than the center of the balloon distal portion. The proximal side of the balloon distal portion means a region closer to the proximal side than the center of the balloon distal portion.
Crystallinity is an index indicating a degree of crystallinity of a resin, and is represented by (crystalline band

intensity (absorbance)/amorphous band intensity (absorbance)) × 100 (%) in the present specification. The crystalline band refers to a band that disappears as confirmed by FT-IR measurement at a melting temperature, and the amorphous band refers to a band that does not disappear as confirmed by FT-IR measurement even at a melting temperature. Here, in a case where there are a plurality of crystalline bands and amorphous bands, one crystalline band and one amorphous band are selected as described below. Specifically, each peak with minimal overlap and baseline drift is selected.

That is, the crystallinity is represented as follows.

[Math. 1]

$$\text{Crystallinity} = \frac{\text{Intensity of specific crystalline band}}{\text{Intensity of specific amorphous band}} \times 100\ [\%] \qquad \text{(Equation 1)}$$

Here, in a case where there is one crystalline band, a specific crystalline band is the one crystalline band, and in a case where there is a plurality of crystalline bands, the specific crystalline band is a band selected as described above. In a case where there is one amorphous band, the specific amorphous band is the one amorphous band, and in a case where there is a plurality of amorphous bands, the specific amorphous band is a band selected as described above.

Furthermore, regarding the crystalline band intensity and the amorphous band intensity, for example, in a case where the resin is a polyamide-based resin (resin containing polyamide or polyamide elastomer), the crystalline band intensity is a crystalline band defined at an intensity of 1161 $cm^{-1}$. Moreover, for example, in a case where the resin is a polyamide-based resin (resin containing polyamide or polyamide elastomer), the amorphous band intensity is an amorphous band intensity defined at an intensity of 1369 $cm^{-1}$.

The crystallinity of the resin is measured as a crystallinity distribution confirmed by imaging IR. In the present specification, the measurement of the crystallinity distribution is carried out under the conditions as follows.

Measurement method: Microscopic-transmission method
Measurement range: 700 μm square (128 pixel square)
Element size: 5.5 μm square
Integration: 128 times
Spectral resolution: 4 $cm^{-1}$
Pretreatment: A sample is embedded in an epoxy resin and processed with a microtome into a thin section having a thickness of about 10 μm.

Furthermore, in the present specification, melting means that at least part of a resin is melted, preferably 80% by mass or more of the resin is melted, more preferably 95% by mass or more of the resin is melted, and still more preferably 99% by mass or more of the resin is melted. A melting temperature is not particularly limited as long as it is a temperature at which the resin melts, but in a case where the resin is a crystalline thermoplastic resin, the melting temperature is preferably a melting point +10°C or higher of the thermoplastic resin, and more preferably a melting point +20°C to 80°C of the thermoplastic resin. In a case where the resin is an amorphous thermoplastic resin, the melting temperature is preferably a glass transition temperature +10°C or higher of the thermoplastic resin, and more preferably a glass transition temperature +20°C to 80°C of the thermoplastic resin. Specifically, for example, the melting temperature is 150°C to 300°C, and 200°C to 250°C.

In the present specification, the "crystallinity on the distal side of the balloon distal portion" is an average value of crystallinity of the balloon in a region closer to the distal side than the center of the balloon distal portion in the cross-sectional view in the axial direction. The "crystallinity on the distal side of the balloon distal portion" is an average value of crystallinity of the balloon in a region closer to the proximal side than the center of the balloon distal portion in the cross-sectional view in the axial direction. The average value of crystallinity can be obtained by calculating an average value in the above-described region. In a case where the balloon includes two or more layers, the crystallinity of the balloon distal portion means a crystallinity of a balloon base layer.

In the balloon catheter according to the suitable embodiment of the present invention, the crystallinity on the distal side of the balloon distal portion is preferably 10% to 50%, more preferably 20% to 45% or less, and may be 30% to 40%. The crystallinity on the proximal side of the balloon distal portion is preferably 10% to 50%, and may be 20% to 45% or 30% to 45%.

In the present specification, it is preferable that the crystallinity of the balloon distal portion gradually decreases from the proximal side toward the distal side. The phrase "gradually decreases" means that in a case where the balloon distal portion is divided into four regions in the axial direction, the average value of crystallinity of the regions is (region present on proximal side ≥ region present on distal side). Since the hardness gradually changes from the distal side to the proximal side as the crystallinity gradually changes, it is possible to form gradient physical properties, contribute to the improvement of passability in a living body, and maintain the flexibility against bending at the balloon distal portion.

In the present specification, it is preferable that the crystallinity on the distal side of the balloon distal portion is 2% to 6% lower than that on the proximal side. The crystallinity of the distal side of the balloon distal portion may be 1.5% to 6%, preferably 1.7% to 5.8%, more preferably 1.8% to 5.5% lower than that on the proximal side. As a result, since the hardness gradually changes from the distal side to the proximal side, it is possible to form gradient physical properties, contribute to the improvement of passability in a living body, and maintain the flexibility against bending at the balloon distal portion.

A balloon catheter 3 according to an embodiment (first embodiment) of the present invention will be described with reference to Figs. 1 to 3A. Fig. 1 is a diagram illustrating an overall configuration of the balloon catheter 3 according to the embodiment of the present invention. Fig. 2 is a diagram illustrating a cross section along the axial direction in the vicinity of the distal end of the balloon catheter 3 according to the embodiment. Fig. 3A is a partially enlarged diagram in the vicinity of the distal end in Fig. 2.

The balloon catheter 3 according to an embodiment is a medical device used to perform a treatment for a stenosis (lesion). In the treatment, an elongated shaft 310 is inserted into a biological organ, and a balloon 60 disposed on the distal side of the shaft 310 is expanded at the stenosis to push and widen the stenosis.

The balloon catheter 3 is configured as a balloon catheter for PTCA expansion used to expand a stenosis of a coronary artery, and for example, a configuration for the purpose of treating and alleviating a stenosis developed in a biological organ such as another blood vessel, a bile duct, a trachea, an esophagus, another digestive tract, a urethra, an ear and nose lumen, or another organ can be adopted, and a configuration as a delivery balloon catheter used for the purpose of transporting a medical instrument such as a stent in a living body can be adopted.

In the description of the present embodiment, the part (left side in Fig. 1) of the balloon catheter 3 that is inserted into a living body is referred to as a distal side, the part of the balloon catheter 3 on which a hub 340 is disposed is referred to as a proximal side, and the direction in which a shaft 310 of the balloon catheter 3 extends is referred to as an axial direction. A distal portion represents a certain range including the distal end (the most distal end) and its periphery, while a proximal portion represents a certain range including the proximal end (the most proximal part) and its periphery.

As illustrated in Fig. 1, the balloon catheter 3 includes the shaft 310 extending in the axial direction, a distal member 320 disposed on the distal side of the shaft 310, the hub 340 disposed on the proximal side of the shaft 310, and the balloon 60 disposed on the outer periphery of the shaft 310.

As illustrated in Fig. 2, the shaft 310 includes the outer tube shaft 311 provided with the lumen 311H, and the inner tube shaft 312 disposed in the lumen 311H of the outer tube shaft 311. In Figs. 2 and 3A, the shaft according to the suitable embodiment of the present invention corresponds to the inner tube shaft 312.

The shaft 310 has a double tube structure in which an inner tube shaft 312 and an outer tube shaft 311 are arranged with a concentric alignment. In the present embodiment, the balloon catheter 3 is of what is called a rapid exchange type provided with a proximal opening (proximal opening of inner tube shaft 312) 312R through which a guide wire 280 is guided out toward the distal portion part of the shaft 310. The inner tube shaft 312 and the outer tube shaft 311 may not be arranged with a concentric alignment.

As illustrated in Figs. 2 and 3A, the inner tube shaft 312 has a tubular shape in which a guide wire lumen 315 through which the guide wire 280 is inserted is formed. As illustrated in Fig. 2, the inner tube shaft 312 has a tapered portion 318 whose diameter decreases from the proximal end toward the distal end in the vicinity of a proximal portion 65 of the balloon 60 in the axial direction. The inner diameter of the inner tube shaft 312 closer to the proximal side than the tapered portion 318 is larger than the inner diameter of the inner tube shaft 312 where X-ray radiopaque markers 270 described later are positioned, and larger than the outer diameter of the inner tube shaft 312 at the portion where the balloon 60 is bonded to the inner tube shaft 312.

A lumen 311H included in the outer tube shaft 311 has a function as a lumen for a pressurizing medium through which a pressurizing medium flows between the outer tube shaft 311 and the inner tube shaft 312.

The hub 340 includes a port 341 connectable in a liquid-tight and air-tight manner to a supplying device such as an indeflator (not illustrated) for supplying a pressurizing medium to the balloon 60. The port 341 of the hub 340 may be, for example, a known Luer taper which a fluid tube or the like can be connected to or separated from. The pressurizing medium (for example, saline, contrast medium, and the like) can flow into the lumen 311H of the outer tube shaft 311 via the port 341 of the hub 340, and is supplied to the balloon 60 through the lumen 311H.

As illustrated in Figs. 2 and 3A, the inner tube shaft 312 includes an outer layer 313 (shaft outer layer) and an inner layer 314 (shaft inner layer) disposed inward in the radial direction of the outer layer 313. As illustrated in Fig. 2, the inner tube shaft 312 has a tapered portion 318 whose diameter decreases from the proximal end toward the distal end in the vicinity of a proximal portion 65 of the balloon 60 in the axial direction. The outer diameter of the inner tube shaft 312 closer to the proximal side than the tapered portion 318 is larger than the outer diameter of the inner tube shaft 312 where X-ray radiopaque markers 270 described later are positioned, and the outer diameter of the inner tube shaft 312 where the X-ray radiopaque markers 270 are positioned is larger than the outer diameter of the inner tube shaft 312 at the portion where the balloon 60 is bonded to the inner tube shaft 312. The inner diameter of the inner tube shaft 312 closer to the proximal side than the tapered portion 318 is larger than the inner diameter of the inner tube shaft 312 where X-ray radiopaque markers 270 described later are positioned, and larger than the outer diameter of the inner tube shaft 312 at the portion where the

balloon 60 is bonded to the inner tube shaft 312.

The average thickness of the shaft inner layer is preferably 0.5 to 49.5 μm, and more preferably 5 to 25 μm.

The average thickness of the shaft outer layer is preferably 0.5 to 10 μm, and more preferably 1 to 5 μm.

In the cross-sectional view in the axial direction as illustrated in Fig. 3A, a distal portion 312A of the inner tube shaft 312 is formed to intrude in an arc outward in the radial direction from an inner surface 320H of the distal member 320. In other words, as illustrated in Fig. 3A, the distal portion 312A of the inner tube shaft 312 extends to be curved outward in the radial direction and toward the distal end of the balloon catheter 3 from the lumen 310H of the shaft 310 to the distal end.

In the cross-sectional view in the axial direction, the outer layer 313 of the inner tube shaft 312 and the distal portion of the inner layer 314 are formed to intrude in an arc outward in the radial direction from the inner surface 320H of the distal member 320. In other words, as illustrated in Fig. 3A, a distal portion 313A of the outer layer 313 and a distal portion 314A of the inner layer 314 extends to be curved outward in the radial direction and toward the distal end of the balloon catheter 3 from the lumen 310H of the shaft 310 to the distal end.

The inner layer 314 of the inner tube shaft 312 has a portion where the crystallinity gradually decreases from the distal portion 314A of the inner layer 314 toward the proximal side in a region where the distal portion 60A of the balloon 60 is bonded to the inner tube shaft. According to this configuration, the bending strength can be improved while the flexibility is maintained in the vicinity of the fusion portion on the distal member side.

As illustrated in Fig. 3A, the distal portion 312A of the inner tube shaft 312 has a wall thickness smaller than that of a portion other than the distal portion 312A because the balloon 60 intrudes therein.

A modification of the distal portion 3A of the balloon catheter 3 of the present embodiment will be described with reference to Fig. 3B. As illustrated in Fig. 3B, in the cross-sectional view in the axial direction, the distal portion 312A of the inner tube shaft 312 is formed to intrude in an arc outward in the radial direction (upward in the same figure) from the inner surface 320H of the distal member 320. In other words, in the cross-sectional view in the axial direction, the distal portion 312A of the inner tube shaft 312 extends to be curved outward in the radial direction and toward the distal side (left side in the same figure) of the balloon catheter 3 from a lumen 30H of the inner tube shaft 312 as a starting point to a most distal end 312D, and extends to be curved outward in the radial direction and toward the proximal side (right side in the same figure) of the balloon catheter 3 from the most distal end 312D. More specifically, the distal portion 312A of the inner tube shaft 312 extends to be curved outward in the radial direction and toward the distal side (left side in the same figure) of the balloon catheter 3 from a site 30H1 of the lumen 30H of the balloon catheter 3 as the starting point to the most distal end 312D. The distal portion 312A of the inner tube shaft 312 extends from the most distal end 312D toward an apex portion 312P while extending toward the proximal side (right side in the same figure) of the balloon catheter 3.

The distal member 320 includes an interposed portion 321 that is interposed between the distal portion 312A of the inner tube shaft 312 and a distal end 60D of the balloon 60. The interposed portion 321 is engaged with the distal portion 312A of the inner tube shaft 312. The apex portion 312P of the distal portion 312A of the inner tube shaft 312 extends toward the proximal direction in the axial direction. As a result, the interposed portion 321 of the distal member 320 is disposed to be interposed between the apex portion 312P of the distal portion 312A of the inner tube shaft 312, which extends like a claw, and the distal end 60D of the balloon 60. The interposed portion 321 of the distal member 320 strengthens the engagement of the distal member 320, the inner tube shaft 312, and the balloon 60. The interposed portion 321 is positioned between the outer layer 313 of the inner tube shaft 312 and an inner layer 66 of the balloon 60, has a thickness gradually decreasing toward the proximal side, and extends to a terminal end 321P.

An inner surface-side proximal portion 320Ha of the distal member 320 is positioned in a section interposed between the inner surface 320H of the distal member 320 and an inner surface 312H of the inner tube shaft 312 in contact with the distal member 320. The thickness of the inner surface-side proximal portion 320Ha in the section increases along a curvature of the inner surface 312H from the site 310H1 of the lumen 310H of the inner tube shaft 312 toward the distal direction. An outer surface-side proximal portion 320Ga of the distal member 320 is positioned in a section interposed between an outer surface 320G of the distal member 320 and an outer surface 60G of the balloon 60. The thickness of the outer surface-side proximal portion 320Ga in the section increases along a curvature of the balloon 60 from a site 30G1 of an outer periphery 30G of the balloon catheter 3 toward the distal direction. The outer surface 320G of the distal member 320 and the outer surface 60G of the balloon 60 form a straight portion without a level difference in the cross-sectional view in the axial direction. The inner surface 320H of the distal member 320 and the inner surface 312H of the inner layer 314 of the inner tube shaft 312 facing the lumen 310H form a straight portion without a level difference in the cross-sectional view in the axial direction. The straight portion formed by the outer surface 320G of the distal member 320 and the outer surface 60G of the balloon 60 is inclined to intersect with respect to the straight portion of the inner surface 312H so as to form an intersection point by extension lines of respective straight portions on the distal side of the balloon catheter 3. The site 30G1 of the outer surface-side proximal portion 320Ga of the distal member 320 is positioned on the distal side in the axial direction with respect to the site 30H1 of the inner surface-side proximal portion 320Ha. The terminal end 321P of the interposed portion 321 is positioned closer to the distal side in the axial direction than the site 30G1 of the outer surface-side proximal portion 320Ga of the distal member 320. The terminal end 321P of the interposed portion 321 is positioned closer to the distal side in the axial direction than the site 30H1 of the inner surface-side proximal portion 320Ha of the distal

member 320.

As illustrated in Fig. 2, the inner tube shaft 312 is provided with two X-ray radiopaque markers 270 indicating the position of the balloon 60 in the axial direction. As the X-ray radiopaque markers 270, for example, a thin metal wire formed of an X-ray radiopaque material, such as a metal such as platinum, gold, silver, iridium, titanium, or tungsten, or an alloy thereof can be used. As the X-ray radiopaque markers 270, a resin material containing powder made of an X-ray radiopaque material may be used.

As illustrated in Fig. 2, the proximal portion 65 of the balloon 60 is bonded to the distal portion 311A of the outer tube shaft 311. As illustrated in Fig. 3A, a distal portion 60A of the balloon 60 is formed to intrude in an arc inward in the radial direction from the outer surface of the distal member 320. In other words, as illustrated in Figs. 3A and 3B, the distal portion 60A of the balloon 60 is formed in a convex shape to be narrowed toward the distal side. The distal end 60D of the distal portion 60A of the balloon 60 is rounded. In a case where a perpendicular line is drawn along the axial direction so as to be brought into contact with the most distal end 60D1 of the distal portion 60A of the balloon 60, the distal member 320, the inner layer 314, the outer layer 313, the distal member 320, the most distal end 60D1 of the distal portion 60A of the balloon 60, and the distal member 320 are positioned in this order in the radial direction from the center axis.

In the cross-sectional view in the axial direction, the distal portion 60A of the balloon 60 includes a wedge portion 60A1 that is wedge-shaped as illustrated in Fig. 3A. The wedge portion 60A1 includes the parallel portion 60A2 parallel to the axial direction. More specifically, in the cross-sectional view in the axial direction, the wedge portion 60A1 inclined substantially linearly with respect to the axial direction is provided on the outer surface of a base layer 67. A parallel portion 60A2 extending in parallel with the axial direction is provided on the inner surface of the base layer 67. The parallel portion 60A2 extends in parallel with the shaft inner layer 314 of the inner tube and the outer layer 313 of the inner tube shaft. A mixed layer 69 of a balloon inner layer 66 and a balloon outer layer 68 is positioned on the distal side at a portion where the wedge portion 60A1 and the parallel portion 60A2 intersect. According to this configuration, since the rigidity gradually transitions from the distal side to the proximal side of the balloon 60, bending at a portion where the balloon 60 is fused to the inner tube shaft 312 is smooth. In the base layer 67 of the distal portion of the balloon, the proximal side of the wedge portion 60A1 has a higher crystallinity than the distal side. As a result, in combination with the inclination of the wedge portion 60A1, passing through a lesion is facilitated while spreading the lesion apart during the insertion into a hard fibrous stenosis. Since the outer layer 68 is laminated on the outer surface of the base layer 67 of the distal portion of the balloon, passing through and intruding into a soft lesion may be facilitated.

As illustrated in Fig. 2, the balloon catheter 3 includes an outer diameter constant portion 3E, a first tapered portion 3F, a second tapered portion 3G, and a third tapered portion 3H in order from the distal side. The inclination of the second tapered portion 3G with respect to the axial direction is larger than the inclination of the first tapered portion 3F. The inclination of the third tapered portion 3H with respect to the axial direction is smaller than the inclination of the second tapered portion 3G. The above-described multi-stage tapered portion facilitates insertion into and passing through a stenosis. Each of the tapered portions described above is formed under the pressure from the direction corresponding to the inclination. The third tapered portion 3H may be an outer diameter constant portion having a substantially constant outer diameter.

The balloon may have a one-layer structure composed of a base layer, a two-layer structure composed of the inner layer and the base layer, which are laminated from the lumen side, and a three-layer structure composed of the inner layer, the base layer (middle layer), and the outer layer from the lumen side, or may have a structure composed of four or more layers. In a case where the balloon has the three-layer structure, the distal portion 60A of the balloon 60 corresponds to the base layer. The crystallinity of the outer layer in the parallel portion 60A2 is preferably lower than the crystallinity of the inner layer. As a result, it is possible to maintain the flexibility against bending in the fusion (bonding) portion.

In the cross section perpendicular to the axis at the axial center portion of the balloon, the cross-sectional area ratio of the base layer of the balloon is preferably 30% to 70%, and more preferably 35% to 60%.

The average thickness of the base layer is preferably 5 to 20 μm. The thickness of the base layer of the balloon may be set to an appropriate thickness from the viewpoint of a balloon diameter, required rupture resistance performance, and passability.

The average thickness of the base layer of the balloon is calculated by conversion with an original tube design dimension and the thickness of the balloon. The average thickness of the base layer of the balloon is an average thickness of the base layer in a membranous main body constituting the balloon.

The average thickness of the outer layer of the balloon is preferably 5 to 15 μm, and more preferably 5 to 10 μm. In a case where the average thickness is within a range of 5 to 15 μm, abrasion resistance against a hard component such as a calcified lesion can be improved, and the procedure can be applied more safely. The average thickness of the outer layer of the balloon is an average thickness of the outer layer in the membranous main body constituting the balloon.

In the cross section perpendicular to the axis at the axial center portion of the balloon, the cross-sectional area ratio of the outer layer is preferably 20% to 50%, and more preferably 25% to 50%. Within this range, both rupture resistance performance and expansion performance of a hard lesion such as a calcified lesion can be achieved.

The average thickness of the inner layer of the balloon is preferably 0.1 to 10 μm, and more preferably 0.1 to 7 μm.

In a case where the average thickness is within a range of 0.1 to 10 μm, the flexibility of the entire balloon and low compliance characteristics are balanced, which is preferable. The average thickness of the inner layer of the balloon is an average thickness of the inner layer in the membranous main body constituting the balloon.

As illustrated in Fig. 3A, a thickness D1 at the distal end 60D of the balloon 60 positioned at the portion fused to the inner tube shaft 312 is equal to or smaller than a thickness D2 of the inner tube shaft 312 at a site P closer to the proximal side than the portion where the balloon 60 is bonded to the inner tube shaft 312, and is larger than a thickness D3 of the inner tube shaft 312 at the portion bonded to the balloon 60. According to this configuration, since the balloon 60 is inserted into the inner tube shaft 312, the thickness of the balloon 60 is incorporated, and an increase in rigidity can be suppressed. That is, the flexibility in the distal portion 3A of the balloon catheter 3 can be maintained.

As illustrated in Fig. 3, the balloon 60 includes the inner layer 66, the base layer 67, and the outer layer 68 in order from the inner side. At the distal end 60D of the balloon 60, the inner layer 66 and the outer layer 68 are mixed with each other to form the mixed layer 69. The mixed layer 69 is a layer in which the balloon inner layer 66 and outer layer 68 are mixed and integrated.

The balloon catheter according to the suitable embodiment of the present invention preferably includes the distal member that is fused to the distal side of the shaft (inner tube shaft) and is more flexible than the shaft, and the crystallinity of each of the proximal side of the distal member and the distal region of the balloon distal portion is preferably less than 40%. Accordingly, since the difference in crystallinity from the adjacent resin is small, the occurrence of cracks such as delamination can be reduced. The lower limit of the crystallinities of the distal side of the distal member and the distal region of the balloon distal portion is not particularly limited, but is usually 10% or more, and may be 20% or more.

As the crystallinity of the proximal portion of the distal member in the cross-sectional view in the axial direction, an average value of crystallinity of the distal member in a region defined by perpendicular lines drawn with respect to the axial direction at the most distal end of the shaft and a position of 200 μm on the distal side away from the most distal end is employed. The average value of crystallinity can be obtained by calculating an average value of crystallinity for each pixel corresponding to the above-described region.

As the crystallinity of the distal region of the balloon distal portion in the cross-sectional view in the axial direction, an average value of crystallinity of the balloon in a region defined by perpendicular lines drawn with respect to the axial direction at the most distal end of the balloon and a position of 150 μm on the proximal side away from the most distal end is employed. The average value of crystallinity can be obtained by calculating an average value of crystallinity for each pixel corresponding to the above-described region. The same method of calculating the average value based on the crystallinity of the pixels corresponding to the region also applies to the below description, and thus will not be repeatedly described.

In the balloon catheter according to the suitable embodiment of the present invention, the thickness at the distal end of the balloon positioned at the portion fused to the shaft (inner tube shaft) is equal to or smaller than the thickness of the shaft at the site closer to the proximal side than the portion where the balloon is bonded to the shaft, and is larger than the thickness of the shaft at the portion bonded to the balloon. According to this configuration, since the balloon is inserted into the shaft, the thickness of the balloon is incorporated, and an increase in rigidity can thus be reduced. That is, the flexibility in the distal portion of the balloon catheter can be maintained.

Next, a balloon catheter 5 according to the embodiment (second embodiment) of the present invention will be described with reference to Fig. 5. Fig. 5 is a diagram of the balloon catheter 5 according to the second embodiment, which corresponds to Fig. 3B.

As illustrated in Fig. 5, the balloon catheter 5 according to the second embodiment includes an inner tube shaft 512, a distal member 520 disposed on the distal side of the inner tube shaft 512, and a balloon 560 disposed on the outer periphery of the inner tube shaft 512.

As illustrated in Fig. 5, the inner tube shaft 512 includes an outer layer 513 and an inner layer 514 disposed inward in the radial direction of the outer layer 513.

In the cross-sectional view in the axial direction, as illustrated in Fig. 5, a distal portion 512A of the inner tube shaft 512 is formed to intrude in an arc outward in the radial direction from an inner surface 520H of the distal member 520. More specifically, the distal portion 512A of the inner tube shaft 512 extends to be curved outward in the radial direction and toward the distal side of the balloon catheter 5 from a site 512H1 of a lumen 512H of the inner tube shaft 512 as the starting point to a most distal end 512D.

At a site 512E positioned on the proximal side from the distal portion 512A of the inner tube shaft 512, the inner tube shaft 512 is configured to inflate outward in the radial direction. According to this configuration, since an inflating site 512F is in such a positional relationship as to be engaged with an interposed portion 521 of the distal member 520 described later, the bonding strength of the inner tube shaft 512 to the distal member 520 is improved.

The distal member 520 includes an interposed portion 521 that is interposed between the distal portion 512A of the inner tube shaft 512 and a distal end 560D of the balloon 560. In other words, the proximal portion 522 of the distal member 520 is positioned between the distal end 560D of the balloon 560 and the distal portion 512A of the inner tube shaft 512. According to this configuration, even though the distal member 520 is bent, the interposed portion 521 functions as a cushioning material between the distal end 560D of the balloon 560 and the distal portion 512A of the inner tube shaft 512.

Thus, it is possible to minimize curling or detachment of the distal end 560D of the balloon 560. It is considered that the interposed portion 521 is formed because the distal member 520 flows between the inner tube shaft 512 and the balloon 560 during molding.

An inner surface-side proximal portion 520Ha of the distal member 520 is positioned in a section interposed between the inner surface 520H of the distal member 520 and an inner surface 512H of the inner tube shaft 512 in contact with the distal member 520. The thickness of the inner surface-side proximal portion 520Ha in the section increases along a curvature of the inner surface 512H from the site 510H1 of the lumen 510H of the inner tube shaft 512 toward the distal direction. The distal portion 512A of the inner tube shaft 512 is positioned between the inner surface-side proximal portion 520Ha of the distal member 520 and the proximal portion 522 (interposed portion 521) of the distal member 520. The distal portion 512A, the inner surface-side proximal portion 520Ha, and the interposed portion 521 are in contact with each other by fusion.

As illustrated in Fig. 5, the distal member 520 is disposed to wrap around the distal end 560D of the balloon 560. The distal member 520 covers the radially outer side of a most distal end 560D1 of the balloon 560 with an outer surface-side proximal portion 520Ga of the distal member 520, and a curved boundary is configured with the outer surface-side proximal portion 520Ga and from the most distal end 560D1 to a site 560G1 of the outer periphery 560G. The distal member 520 covers the radially inner side of the most distal end 560D1 with a middle proximal portion 522M of the distal member 520, and defines a curved boundary with the middle proximal portion 522M from the most distal end 560D1 toward the proximal side. In other words, the most distal end 560D1 of the distal end 560D of the balloon 560 is covered with the outer surface-side proximal portion 520Ga and the middle proximal portion 522M of the distal member 520. According to this configuration, a bonded area between the distal member 520 and the distal portion 560A of the balloon 560 is increased, and the curling of the distal end 560D of the balloon 560 is minimized, so that the bonding strength is improved.

As illustrated in Fig. 5, the distal portion 560A of the balloon 560 is formed to intrude in an arc inward in the radial direction from the outer surface of the distal member 520.

As illustrated in Fig. 5, the distal end 560D of the balloon 560 is formed in a tongue shape toward the distal side of the balloon catheter 5. The distal end 560D of the balloon 560 is configured to have a thickness decreasing toward the distal side of the site 562, and is configured to have a thickness increasing toward the proximal side of the site 562.

The balloon 560 includes an inner layer 566, a base layer 567, and an outer layer 568 similarly to the balloon 60 according to the first embodiment described above. At the distal portion 560A of the balloon 560, the inner layer 566 and the outer layer 568 are mixed with each other to form a mixed layer 569.

Next, a balloon catheter 6 according to the embodiment (third embodiment) of the present invention will be described with reference to Fig. 6. Fig. 6 is a diagram of the balloon catheter 6 according to the third embodiment, which corresponds to Fig. 3B.

As illustrated in Fig. 6, the balloon catheter 6 according to the third embodiment includes an inner tube shaft 612, a distal member 620 disposed on the distal side of the inner tube shaft 612, and a balloon 660 disposed on the outer periphery of the inner tube shaft 612.

As illustrated in Fig. 6, the inner tube shaft 612 includes an outer layer 613 and an inner layer 614 disposed inward in the radial direction of the outer layer 613.

In the cross-sectional view in the axial direction, as illustrated in Fig. 6, a distal portion 612A of the inner tube shaft 612 is formed to intrude in an arc outward in the radial direction from an inner surface 620H of the distal member 620. More specifically, the distal portion 612A of the inner tube shaft 612 extends to be curved outward in the radial direction and toward the distal side of the balloon catheter 6 from a site 612H1 of a lumen 612H of the inner tube shaft 612 as the starting point to a most distal end 612D.

The distal member 620 includes an interposed portion 621 that is interposed between the distal portion 612A of the inner tube shaft 612 and a distal end 660D of the balloon 660. In other words, the proximal portion 622 of the distal member 620 is positioned between the distal end 660D of the balloon 660 and the distal portion 612A of the inner tube shaft 612. According to this configuration, even though the distal member 620 is bent, the interposed portion 621 functions as a cushioning material between the distal end 660D of the balloon 660 and the distal portion 612A of the inner tube shaft 612. Thus, it is possible to minimize curling or detachment of the distal end 660D of the balloon 660. It is considered that the interposed portion 621 is formed because the distal member 620 flows between the inner tube shaft 612 and the balloon 660 during molding. The interposed portion 621 according to the third embodiment is formed in a shorter length in the axial direction than the interposed portion 521 according to the second embodiment.

As illustrated in Fig. 6, the distal member 620 is disposed to wrap around the distal end 660D of the balloon 660. The distal member 620 covers the radially outer side of a most distal end 660D1 of the balloon 660 with an outer surface-side proximal portion 620Ga of the distal member 620, and a curved boundary is configured with the outer surface-side proximal portion 620Ga and from the most distal end 660D1 to a site 660G1 of the outer periphery 660G. The distal member 620 covers the radially inner side of the most distal end 660D1 with a middle proximal portion 622M of the distal member 620, and defines a curved boundary with the middle proximal portion 622M from the most distal end 660D1 toward the proximal side. In other words, the most distal end 660D1 of the distal end 660D of the balloon 660 is covered with the

outer surface-side proximal portion 620Ga and the middle proximal portion 622M of the distal member 620. According to this configuration, since the curling of the distal end 660D of the balloon 660 is minimized, the bonding strength is improved. Furthermore, according to this configuration, the bonded area between the distal member 620 and the distal end 660D of the balloon 660 is increased, and the bonding strength is improved.

As illustrated in Fig. 6, the distal portion 660A of the balloon 660 is formed to intrude in an arc inward in the radial direction from the outer surface of the distal member 620. As illustrated in Fig. 6, the distal end 660D of the balloon 660 is formed in a tongue shape toward the distal side of the balloon catheter 6.

The balloon 660 includes an inner layer 666, a base layer 667, and an outer layer 668 similarly to the balloon 60 according to the first embodiment described above. At the distal portion 661 of the balloon 660, the inner layer 666 and the outer layer 668 are mixed with each other to form a mixed layer 669.

Materials of the distal member, the shaft (inner tube shaft), and the balloon, which are components of the balloon catheter according to the suitable embodiment of the present invention, will be described.

Examples of the resin forming the distal member include polyolefins (for example, polyethylene, polypropylene, polybutene, ethylene-propylene copolymer, ethylene-vinyl acetate copolymer, ionomer, or a mixture of two or more types thereof), polyvinyl chloride, polyamide, polyamide elastomer, polyurethane, polyurethane elastomer, polyimide, and fluororesin. The above-described resin may be used singly or in combination of two or more types thereof. The resin forming the distal member is preferable to contain a polyamide elastomer having high flexibility and a small affinity with an adjacent member and a small difference in hardness. The content of the polyamide elastomer in the resin contained in the distal member is preferably 50% by weight or more, more preferably 80% by weight or more, and still more preferably 100% by weight (consisting of a polyamide elastomer). The polyamide elastomer may be used singly or in combination of two or more types thereof.

The polyamide elastomer is a thermoplastic resin composed of a copolymer having a hard segment derived from a polymer that is crystalline and has a high melting point and a soft segment derived from a polymer that is amorphous and has a low glass transition temperature, the polyamide elastomer having amide bonds (-CONH-) in the polymer backbone forming the hard segment. Constituent units having amide bonds (-CONH-) in the polymer backbone forming the hard segment are also referred to as amide units of the polyamide elastomer.

The "amide units of the polyamide elastomer" in the present specification refers to repeating units derived from amide bonds in the polymer chain of the polyamide elastomer, and the amide units in the polyamide elastomer are preferably at least one of repeating units represented by Formula (1) or Formula (2) below.

[Chem. 1]

Formula (1)

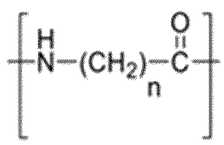

In Formula (1), n is preferably an integer of 2 to 20, and more preferably an integer of 5 to 11.

[Chem. 2]

Formula (2)

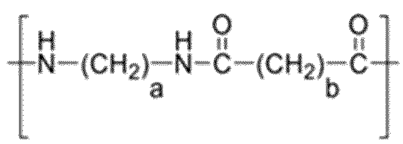

In Formula (2), a is preferably an integer of 4 to 12, b is preferably an integer of 4 to 10, a is more preferably an integer of 11 or 12, and b is more preferably an integer of 4 to 8.

Therefore, the polyamide elastomer preferably contains at least one of the repeating units represented by Formula (1) or Formula (2).

Furthermore, as the polyamide elastomer, a polyamide block copolymer represented by Formula (3) or (4)

described in the section for the shaft outer layer may be used singly or in combination of two or more types thereof.

Examples of the polymer forming the soft segment include polyester and polyether. Moreover, examples thereof include polyethers such as polyethylene glycol, polypropylene glycol, polytetramethylene ether glycol (PTMG), and polyester polyol, and ABA-type triblock polyether diols. These can be used singly or in combination of two or more types thereof. Furthermore, a polyether diamine or the like obtained by reacting ammonia or the like with the terminal of polyether can be used, and for example, an ABA-type triblock polyether diamine can be used.

The content of the soft segment in the polyamide elastomer is preferably 1% to 50% by weight, more preferably 1% to 35% by weight, and still more preferably 10% to 30% by weight.

Among these, the polyamide elastomer is preferably a polyether block amide copolymer formed by condensation polymerization of a polyether block amide in which a polyamide block as a hard segment and a polyether block as a soft segment are bonded via an ester bond.

As the polyamide elastomer, a chain extender such as a dicarboxylic acid may be used in addition to the hard segment and the soft segment.

These polyamide elastomers may be used singly or in combination of two or more types thereof.

The Shore D hardness of the polyamide elastomer used for the distal member is preferably 50 to 80 and more preferably 55 to 80 from the viewpoint of flexibility. As a method of measuring hardness of the polyamide elastomer, the Shore D hardness according to ISO 868 is used.

The polyamide elastomer may be synthesized, or a commercially available product may be used. Examples of the commercially available product include PEBAX (registered trademark) series such as PEBAX (registered trademark) 2533, 3533, 4033, 5533, 6333, 7033, and 7233 manufactured by Arkema Inc., VESTAMID (registered trademark) E series manufactured by Polyplastics-Evonik Corporation, Grilflex ELG5660 (manufactured by EMS-CHEMIE AG, polyamide block ratio: 67% by weight, Shore D hardness: 56), and Grilflex ELG6260 (manufactured by EMS-CHEMIE AG, product name: Grilflex, polyamide block ratio: 85% by weight, Shore D hardness: 62). These commercially available products may be mixed and used.

The distal member can contain a pigment or dye that develops white, black, blue, red, or yellow, and a mixture thereof. Such a pigment or dye may be selected from materials that absorb a laser beam and generate heat. Examples of a material that absorbs a laser beam and generates heat (hereinbelow, also referred to as a laser beam-absorbing material) include carbon black, activated carbon, graphite, carbon nanotube, and fullerene; and condensed polycyclic organic pigments such as cyanine-based pigments, nickel dithiolene-based pigments, squarylium-based pigments, naphthoquinone-based pigments, diimmonium-based pigments, azo-based organic pigments, phthalocyanine-based pigments, naphthalocyanine-based pigments, and azulenocyanine-based pigments.

Furthermore, the distal member can be configured to include a powdered contrast medium. Specific examples of the material include compounds of gold, titanium, bismuth, and tungsten. Moreover, for the distal member, a reinforcing body made of tungsten, SUS, or the like may be disposed in the above-described material. Examples of a mode of the reinforcing body include a coil shape and a blade shape.

Examples of the resin constituting the shaft (inner tube shaft) include polyolefins such as polypropylene, ethylene-propylene copolymer, and ethylene-vinyl acetate copolymer, thermoplastic resins such as modified polyolefin resin, soft polyvinyl chloride, various elastomers such as polyurethane elastomer, polyamide elastomer, and polyester elastomer, and crystalline plastics such as polyamide, crystalline polyethylene, and crystalline polypropylene.

Among these, the resin constituting the shaft (inner tube shaft) is preferably a polyamide and/or a polyamide elastomer. In a further suitable form, the resin constituting the shaft outer layer contains a polyamide elastomer, and the resin constituting the shaft inner layer contains a polyamide. In a further suitable form, the resin constituting the outer layer contains a polyamide elastomer and a polyamide, and the resin constituting the shaft inner layer contains a polyamide. In a further suitable form, the resin constituting the shaft outer layer contains a polyamide elastomer and a polyamide, and the resin constituting the shaft inner layer is composed of a polyamide (alone). Since such a form is employed, the interlayer adhesion between the inner and outer layers of the shaft is improved. The outer layer preferably has an inner layer containing a polyamide elastomer formed in close contact with a surface, and the inner layer is preferably formed in close contact with the entire surface of the outer layer.

A mixing weight ratio in a case where the shaft outer layer contains a polyamide elastomer and a polyamide is not particularly limited, but in consideration of the rigidity of the catheter shaft and the fusion affinity with the inner layer, polyamide elastomer : polyamide = 1 : 0.1 to 1 : 10 is preferable, and 1 : 0.5 to 1 : 5 is more preferable.

The polyamide that can be contained in the shaft inner layer and the outer layer is not particularly limited as long as it is a polymer having an acid amide bond (-CO-NH-) in the backbone, and is usually produced by polymerization of lactam or amino acid having a ring structure or condensation polymerization of dicarboxylic acid and diamine. Therefore, as this polyamide, a homopolyamide is preferably used. Examples of a homopolymerizable monomer include ε-caprolactam, aminocaproic acid, enantholactam, 7-aminoheptanoic acid, 11-aminoundecanoic acid, 12-aminododecanoic acid, 9-aminononanoic acid, and piperidone.

In the condensation polymerization of a dicarboxylic acid and a diamine, examples of the dicarboxylic acid

include adipic acid, sebacic acid, dodecane dicarboxylic acid, glutaric acid, terephthalic acid, 2-methylterephthalic acid, isophthalic acid, and naphthalenedicarboxylic acid. Examples of the diamine include tetramethylenediamine, hexamethylene diamine, nonamethylene diamine, decamethylene diamine, undecamethylene diamine, dodecamethylene diamine, p-phenylene diamine, and m-phenylenediamine.

Examples of the polyamide include NYLON 4, 6, 7, 8, 11, 12, 6.6, 6.9, 6.10, 6.11, 6.12, 6T, 6/6.6, 6/12, 6/6T, and 6T/6I. The polyamide can be used singly or in combination of two or more types thereof. Among these, the polyamide is preferably NYLON 11 or NYLON 12 (for example, Grilamid L16 and L25 manufactured by EMS-CHEMIE AG, and DIAMID L1940 and L1940W manufactured by Polyplastics-Evonik Corporation).

The terminal of the polyamide may be capped with a carboxyl group, amine, or the like. Examples of the carboxylic acid include aliphatic monocarboxylic acids such as adipic acid, caproic acid, caprylic acid, capric acid, lauric acid, myristic acid, palmitic acid, stearic acid, and behenic acid. Furthermore, examples of the amine include aliphatic primary amines such as hexylamine, octylamine, decylamine, laurylamine, myristylamine, palmitylamine, stearylamine, and behenylamine.

The weight-average molecular weight of the polyamide is preferably $2.0 \times 10^4$ to $5.0 \times 10^4$, more preferably $3.0 \times 10^4$ to $5.0 \times 10^4$, and still more preferably $4.0 \times 10^4$ to $5.0 \times 10^4$.

The molecular weight of the polymer according to the suitable embodiment of the present invention can be measured by a known method such as mass spectrometry, light scattering, liquid chromatography, gas chromatography, or gel filtration chromatography (GPC), and in the present specification, the molecular weight is measured by GPC.

As the polyamide elastomer that can be contained in the shaft outer layer, the polyamide elastomer described in the section of the above-described distal member can be used.

The weight of the amide units of the polyamide elastomer capable of constituting the shaft outer layer is preferably 60% by weight or more, and more preferably 60% to 85% by weight.

The sentence "the weight of the amide units of the polyamide elastomer is 60% by weight or more" as used herein means that the weight of the amide units (alternatively, it is also referred to as repeating units derived from amide bonds) is 60% by weight or more with respect to the total weight of the polyamide elastomer in the polymer chain of the polyamide elastomer.

In the method of calculating the weight of the amide units of the polyamide elastomer, a peak derived from the amide units in the polyamide elastomer is specified using $^1$H-NMR and $^{13}$C-NMR, a unit ratio of the amide units in one polymer chain is calculated from an integral ratio of $^1$H-NMR, and a value obtained by multiplying the unit ratio by a molecular weight of the amide units is then defined as % by weight of the amide units.

The polyamide elastomer that can be contained in the shaft outer layer is preferably a polyamide block copolymer, and more preferably a binary block copolymer. The polyamide block copolymer preferably contains 60% by weight or more of an amide unit represented by Formula (1) or Formula (2), which is the amide unit of the polyamide elastomer, with respect to the weight (100% by weight) of one polymer chain of the polyamide block copolymer.

The polyamide block copolymer more preferably contains at least one selected from the group consisting of Formula (3) and Formula (4) below.

[Chem. 3]

Formula (3)

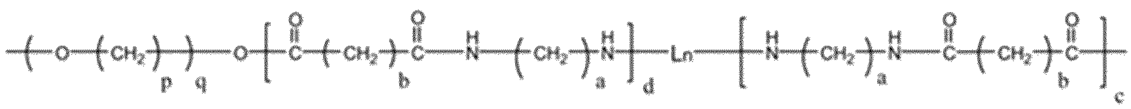

(In Formula (3), a is an integer of 4 to 12, b is an integer of 4 to 10, c and d are each an integer of 0 to 100, p is an integer of 2 to 4, and q is an integer of 1 to 100,

where, Ln is a linker moiety and is -C(O)-R-C(O)-, and
R is an alkylene group consisting of 2 to 12 methylene groups and is preferably an alkylene group consisting of 4 to 10 methylene groups.)

[Chem. 4]

Formula (4)

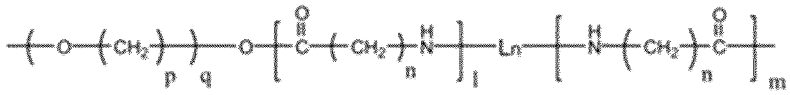

(In Formula (4), n is an integer of 5 to 11, m and 1 are each an integer of 0 to 100, p is an integer of 2 to 4, and q is an integer of 1 to 100,

where, Ln is a linker moiety and is -C(O)-R-C(O)-, and
R is an alkylene group consisting of 2 to 12 methylene groups and is preferably an alkylene group consisting of 4 to 10 methylene groups.)

Furthermore, in the above-described formulae, the alkylene group having 2 to 12 methylene groups at R may be linear, branched, or cyclic, but is not particularly limited, and specific examples thereof include a tetramethylene group, a 2-methylpropylene group, a 1,1-dimethylethylene group, a n-pentylene group, a n-hexylene group, a n-nonylene group, a 1-methyloctylene group, a 6-methyloctylene group, a 1-ethylheptylene group, a 1-(n-butyl)pentylene group, a 4-methyl-1-(n-propyl)pentylene group, a 1,5,5-trimethylhexylene group, a 1,1,5-trimethylhexylene group, a n-decylene group, a 1-methylnonylene group, a 1-ethyloctylene group, a 1-(n-butyl)hexylene group, a 1,1-dimethyloctylene group, a 3,7-dimethyloctylene group, an n-undecylene group, and a 1-methyldecylene group.

Therefore, in the polyamide elastomer that can be used for the shaft outer layer, the polyamide block copolymer represented by Formula (3) or (4) described above may be used singly or in combination of two or more types thereof.

Furthermore, the terminal of the polyamide block polymer may be capped with a carboxyl group, amine, or the like. Examples of the carboxylic acid include aliphatic monocarboxylic acids such as adipic acid, caproic acid, caprylic acid, capric acid, lauric acid, myristic acid, palmitic acid, stearic acid, and behenic acid. Furthermore, examples of the amine include aliphatic primary amines such as hexylamine, octylamine, decylamine, laurylamine, myristylamine, palmitylamine, stearylamine, and behenylamine.

The weight-average molecular weight of the polyamide elastomer is preferably $1.0 \times 10^4$ to $1.0 \times 10^5$, more preferably $2.0 \times 10^4$ to $5.0 \times 10^4$, and still more preferably $2.0 \times 10^4$ to $4.0 \times 10^4$.

The Shore D hardness of the polyamide elastomer is preferably 54 or more and 62 or less from the viewpoint of flexibility.

The above-described polyamide elastomer may be synthesized, or a commercially available product may be used. Examples of the commercially available product include Grilflex ELG5660 (manufactured by EMS-CHEMIE AG, polyamide block ratio: 67% by weight, Shore D hardness: 56) and Grilflex ELG6260 (manufactured by EMS-CHEMIE AG, product name: Grilflex, polyamide block ratio: 85% by weight, Shore D hardness: 62).

For the shaft inner layer, a modified polyolefin resin can also be used in addition to the polyamide-based resin described above. Alternatively, a modified polyolefin resin may be used for the shaft inner layer without using the polyamide-based resin. Examples of the modified polyolefin resin include polyethylene, polypropylene, an $\alpha$-olefin (for example, 1-butene, 1-hexene, 4-methyl-1-pentene, 1-octene, 1-decene, and the like) polymer, an ethylene-propylene copolymer, a cycloolefin polymer (for example, a cyclic olefin polymer such as norbornene, cyclobutene, or cyclopentene), a cycloolefin copolymer (for example, a copolymer of a cyclic olefin and a chain olefin such as polyethylene, or a copolymer of a cyclic olefin and a diene such as 1,4-hexadiene), and mixtures thereof, which are materials with pendants containing polar and reactive groups; an ethylene-vinyl acetate copolymer. The copolymer has no particular limitation on the structure thereof and may be any one of a random copolymer, an alternating copolymer, a periodic copolymer, or a block copolymer. Among these, the modified polyolefin resin is preferably a modified polyethylene (such as high-density polyethylene, low-density polyethylene, or linear low-density polyethylene) or a modified polypropylene, and more preferably a modified polyethylene, from the viewpoint of the sliding properties of a modified polyolefin layer.

Examples of the polar group and reactivity described above include a carboxyl group, a carboxylic anhydride group, a hydroxyl group, an alkoxy group, an imide group, an acryloyl group, a methacryloyl group, a silanyl group, and/or a silanol group.

As the modified polyolefin resin, a commercially available product may be used, and examples thereof include MODIC (registered trademark) series (H511, H503, L502, L533, L504, M142, M502, M512, M545, A543, A515), LINKLON (registered trademark) series (all of which are manufactured by Mitsubishi Chemical Corporation), Admer (registered trademark) series (manufactured by Mitsui Chemicals, Inc.), and HIMILAN (registered trademark) series (Du Pont-Mitsui Polychemicals Co., Ltd.).

The weight-average molecular weight of the modified polyolefin resin is preferably 1,000 to 10,000,000.

In a case where a modified polyolefin resin is used for the shaft inner layer, the resin constituting the shaft outer layer preferably contains a polyamide and/or a polyamide elastomer. In a further suitable form, the resin constituting the shaft outer layer is preferably a polyamide and/or a polyamide elastomer. As another suitable form, the resin constituting the shaft outer layer preferably contains a polyamide and a polyamide elastomer. As another suitable form, it is preferable that a middle layer is provided between the inner layer containing the modified polyolefin resin and the outer layer, the middle layer and the outer layer contain a polyamide, and the outer layer contains a laser beam-absorbing material described later. Since such a form is employed, the effects of the outer layer such as an improvement in abrasion resistance, a protection of the strength layer from external damage, and minimizing pinholes are easily exhibited, and the effects of the inner layer such as ensuring the required pressure resistance strength and compliance are also easily exhibited. Furthermore, since such a form is employed, the interlayer adhesion between the inner and outer layers of the shaft is improved.

It is also possible to improve the pressure resistance strength by applying a composite material containing an inorganic compound to the shaft inner layer.

The shaft (specifically, shaft inner layer) can contain a pigment or dye that develops white, black, blue, red, or yellow, and a mixture thereof. Such a pigment or dye may be selected from materials that absorb a laser beam and generate heat. Examples of a material that absorbs a laser beam and generates heat (laser beam-absorbing material) include carbon black, activated carbon, graphite, carbon nanotube, and fullerene; and condensed polycyclic organic pigments such as cyanine-based pigments, nickel dithiolenebased pigments, squarylium-based pigments, naphthoquinonebased pigments, diimmonium-based pigments, azo-based organic pigments, phthalocyanine-based pigments, naphthalocyanine-based pigments, and azulenocyanine-based pigments. In a suitable aspect, the shaft inner layer contains a laser beam-absorbing material and the shaft outer layer is substantially free of the laser beam-absorbing material. According to such an aspect, when the inner layer absorbs a laser beam and generates heat, the temperature thereof increases to be higher than the glass transition point, deformation associated with pressurization is accelerated, and furthermore, the temperature reaches the melting point to increase fluidity, and the form of "the distal portion of the inner layer intrudes in an arc outward in the radial direction from the inner surface of the distal member, and the distal portion of the outer layer intrudes in an arc inward in the radial direction from the outer surface of the distal member" is thus easily achieved. The blending amount of the laser beam-absorbing material is not particularly limited, but may be, for example, 1% to 25% by weight, 1% to 20% by weight, 1% to 10% by weight, or 2% to 8% by weight in the inner layer. In a case where the laser beam-absorbing material is blended in the shaft inner layer, it is preferable to select a transparent shaft outer layer containing the resin described above. Accordingly, the inner layer easily achieves the above-described form.

As necessary, the surface of the outermost layer capable of coming into contact with a living body may be coated with a hydrophilic lubricating material. The hydrophilic lubricating material is not particularly limited as long as it is hydrophilic and has a lubricating property, and a known material can be used. Specific examples thereof include copolymers of epoxy groupcontaining monomers such as glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether with hydrophilic monomers such as N-methylacrylamide, N,N-dimethylacrylamide, and acrylamide; (co)polymers containing the above-described hydrophilic monomer; cellulose-based polymer substances such as hydroxypropyl cellulose and carboxymethyl cellulose; polysaccharides, polyvinyl alcohol, methyl vinyl ether-maleic anhydride copolymers, water-soluble polyamides, poly(2-hydroxyethyl (meth)acrylate), polyethylene glycol, polyacrylamide, polyvinylpyrrolidone, and copolymers of polyvinylpyrrolidone and polyurethane described in US 4,100,309 and JP S59-19582 A. These hydrophilic lubricating materials may be used singly or in the form of a mixture of two or more types thereof.

Furthermore, as necessary, the surface of the outer layer containing the polyamide elastomer formed on the outermost layer capable of coming into contact with a living body may be further coated with a biocompatible material or an antithrombotic material. As the biocompatible material and the antithrombotic material, various known polymers can be used singly or in combination, and for example, natural polymers (such as collagen, gelatin, chitin, chitosan, cellulose, polyaspartic acid, polyglutamic acid, and polylysine, casein) and synthetic polymers (such as phospholipid polymers, methacryloyloxyethyl phosphorylcholine (MPC) block polymers with a phosphate group in the side chain, polyhydroxyethyl methacrylate, copolymer of hydroxyethyl methacrylate and styrene (for example, HEMA-St-HEMA block copolymer), polymethyl methacrylate, polylactic acid, polyglycolic acid, lactic acid-glycolic acid copolymer, polyethylene, and polypropylene) can be suitably used.

The base layer of the balloon preferably contains a polyamide. Furthermore, in the base layer containing a polyamide, the polyamide accounts for preferably 50% by weight or more, more preferably 80% by weight or more, most preferably 100% by weight (that is, consisting of a polyamide resin) in a resin constituting the inner layer. In a case where polyamide is contained as the resin of the base layer, pressure resistance strength and low compliance characteristics required during expansion of the catheter balloon can be ensured, which is preferable. The base layer may contain a known additive as necessary, or may be composed only of a polyamide.

As the polyamide, those described in the section of the above-described distal member can be appropriately selected and used.

The polyamide used for the base layer of the balloon can be used singly or in combination of two or more types thereof.

Among these, the polyamide used for the base layer of the balloon is preferably NYLON 11 or NYLON 12 (for example, Grilamid L16 and L25 manufactured by EMS-CHEMIE AG).

The weight-average molecular weight of the polyamide used for the base layer of the balloon is preferably $2.0 \times 10^4$ to $5.0 \times 10^4$, more preferably $3.0 \times 10^4$ to $5.0 \times 10^4$, and still more preferably $4.0 \times 10^4$ to $5.0 \times 10^4$.

Examples of the additive contained in the base layer as necessary include higher alcohols, hydroxybenzoic acid esters, and aromatic sulfonamides, but are not necessarily limited thereto.

The outer layer of the balloon preferably contains an elastomer. Moreover, the inner layer of the balloon and the balloon outer layer preferably contain an elastomer. The outer layer containing an elastomer may contain 1% to 100% by weight of an elastomer, and may contain a known additive as necessary, or may be composed only of the elastomer. In a case where the balloon is attached to the catheter and inserted into a body, with the outer layer containing an elastomer formed on the outermost layer, the balloon is flexible and thus excellent in passability through a blood vessel or a lumen in a living body.

Additives that may be optionally included in the outer layer of the balloon are the same as those for the base layer of the balloon, and thus are not described herein.

The inner layer of the balloon preferably contains a polyamide elastomer because of good adhesiveness with the base layer of the balloon and resistance to delamination. Furthermore, in the inner layer containing a polyamide elastomer, the polyamide elastomer accounts for preferably 50% by weight or more, more preferably 80% by weight or more, most preferably 100% by weight (that is, consisting of a polyamide elastomer resin) in a resin constituting the inner layer.

In the balloon, in a case where the inner layer containing a polyamide elastomer is formed on the innermost side, it is possible to provide a catheter balloon exhibiting stable pressure resistance and to impart flexibility to the entire balloon, resulting in ensuring excellent passability in a blood vessel or a body cavity.

In a case where the inner layer containing a resin of the same series as the outer layer of the shaft is formed on the innermost side of the balloon, the adhesive affinity of both resins is excellent, and the fusion bonding strength can be maintained high. In the case where the inner layer containing a polyamide elastomer is formed on the innermost side and the outer layer containing a polyamide or a polyamide elastomer is formed on the shaft, affinity between both is enhanced, and the bonding strength is excellent, which are preferable.

As the polyamide elastomer contained in the inner layer and outer layer of the balloon, the same materials as the polyamide elastomer in the above-described shaft can be used. Thus, the description of the polyamide elastomer will not be repeated herein.

As described above, a preferable aspect of the balloon according to the present invention preferably includes a membranous main body provided with an inner layer that contains a polyamide elastomer and is provided on the innermost side, a base layer that contains a polyamide and is laminated on a surface of the inner layer, and an outer layer that contains a polyamide elastomer and is further provided on the outer side of the base layer. As a result, it is possible to provide the balloon exhibiting the excellent low compliance characteristics without impairing the balance between pressure resistance performance and passability performance.

In the balloon according to the suitable embodiment of the present invention, the base layer, the outer layer, and the inner layer are all preferably transparent.

<Method of Manufacturing Balloon Catheter>

Hereinafter, a preferable embodiment of a method of manufacturing the balloon catheter according to the present invention will be described.

The balloon catheter according to the suitable embodiment of the present invention can be manufactured by producing a bonded body of the distal member and the shaft and fusing the bonded body and the balloon.

The bonded body of the distal member and the shaft is manufactured by bonding the proximal portion part of the distal member and the distal portion part of the shaft. Specifically, it is preferable to include the following steps of: (1) inserting a contact portion between a distal member and a shaft into a hollow portion of an elastic body (step (A)), (2) performing pressurization inward in a radial direction and heating (step (B)), and (3) releasing the pressurization and taking out a bonded body with the distal member and the shaft fused (bonded) (step (C)).

The bonded body and the balloon core material can be fused by adopting the same steps (A) to (C) as described above through the bonded body and the balloon.

Hereinafter, the description will be given with reference to Fig. 4.

(Step (A))

The shaft 10 and the distal member 20 are inserted through a core material 150. As a result, the shaft 10 and the distal member 20 can move and rotate integrally with the core material 150. The core material is made of, for example, metal.

Next, in a state where the rotation axes of the core material 150 and an elastic body 130 are aligned with each other, any one of the core material 150 or the elastic body 130 is moved in the axial direction to dispose the contact portion between the shaft 10 and the distal member 20 in the hollow portion of the elastic body 130. In this state, the shaft 10 and the distal member 20 are not in contact with the hollow portion of the elastic body 130 in the radial direction, and there is a gap. The elastic body has laser transmission properties, and specific examples of a material of the elastic body include silicone rubber and fluororubber. Since the elastic body is used, the elastic body can be elastically deformed in the radial direction by pressurization with a pressurizing member described later to maintain a contact state between the shaft and the distal member, thereby efficiently performing the bonding. Furthermore, since the elastic body has laser transmission properties, the elastic body is not thermally shrunk by laser beam, and thus can be reused.

(Step (B))

Next, the contact portion between the shaft and the distal member is pressurized in the radial direction. The pressurization method is not particularly limited, but for example, a hollow cylindrical pressurizing member can be used. Similar to the core material and the elastic body, the hollow cylindrical pressurizing member is configured to be rotatably supported with the axial direction as a rotation axis. Then, the elastic body can be inserted into the hollow portion of the pressurizing member having such a shape. In this case, the elastic body can be inserted into the hollow portion of the pressurizing member by extending the elastic body in the axial direction so that the diameter of the elastic body is smaller than the inner diameter of the pressurizing member. Moreover, after the elastic body is disposed in the hollow portion of the pressurizing member, the extension in the axial direction is released. The inner diameter of the pressurizing member is preferably smaller than the diameter (outer diameter) of the elastic body. Since the outer diameter of the elastic body and the inner diameter of the pressurizing member are designed in this manner, the elastic body comes into contact with the pressurizing member when the extension is released, and the external pressure is applied inward in the radial direction. The form of applying the external force is not limited to the above-described form, and for example, the elastic body may be pressurized in the radial direction by bringing a rectangular parallelepiped pressurizing member into contact with the elastic body in the radial direction. As another aspect, the elastic body is pressurized in the radial direction by reducing an inner volume of the hollow portion of the pressurizing member. As a result, substantially uniform force can be applied from the inner periphery of the pressurizing member to the outer periphery of the catheter shaft and the distal member through the elastic body.

As described above, since the pressure is applied using the elastic body and the pressurizing member during the heating without applying the pressure during the setup, it is possible to perform highly accurate alignment, and effects such as improvement in quality of fusion and improvement in reproducibility are exerted. Furthermore, since there is a clearance between a workpiece and the elastic body, fusion processing can be performed without affecting the workpiece when the workpiece is inserted into the elastic body. Moreover, imparting a desired shape to the elastic body or the pressurizing member in advance enables the fusion portion of the workpiece to be formed into a tapered shape, a two-stage tapered shape, a gentle round shape, a local constricted portion, or the like.

The shape of the pressurizing member is not limited to the hollow cylindrical shape, and is not particularly limited as long as it can pressurize the elastic body in the radial direction. The pressurizing member has laser transmission properties, similar to the elastic body. The term "laser transmission properties" means that the transmittance of the laser beam is 80% or more with respect to a thickness of 1 mm in the radial direction. A material used for the pressurizing member may be any material that can apply an external force to the elastic body, and examples thereof include glass, quartz, and sapphire.

Next, the contact portion is irradiated with the laser beam while the external force is applied. Since the pressurizing member and the elastic body have laser transmission properties, the contact portion is directly heated. Furthermore, the contact portion can be locally heated by locally irradiating the contact portion with the laser beam emission. The core material 150, the elastic body 130, and the pressurizing member (not illustrated) may rotate about the axial direction as a rotation axis during the heating to cause the distal member 20 and the shaft 10 to be rotated about the axial direction as a rotation axis, so that the heating is uniformly performed.

The laser beam is locally emitted to end face portions and peripheral portions of the two members in a state where both members are arranged in the axial direction. Such laser beam emission causes heat generation of the catheter shaft and/or the distal member themselves and further causes heat transfer from the core material to the catheter shaft, and the contact portion between the proximal side of the distal member and the distal side of the catheter shaft is thus fused to form a fusion portion. A laser beam-absorbing material is preferably contained in at least one of the catheter shaft and/or the

distal member, and preferably at least the catheter shaft. With such a configuration, the member containing the laser beam-absorbing material is locally heated. Furthermore, since the peripheral elastic body has laser transmission properties, the elastic body is not heated, and heat is dissipated to the elastic body and the periphery (for example, core material) from the moment when the fusion portion is heated. Moreover, when the laser beam emission is stopped after fusion, the heat dissipation proceeds, and the fusion portion is cooled. It is considered that such localized rapid heating followed by rapid cooling makes it difficult for crystals to grow, and the crystallinity of the fusion portion thus decreases.

Regarding the laser beam emission, a laser beam having a wavelength that causes the fusion portion to generate heat by radiation heating is emitted. The spot diameter of the laser beam can be set to φ0.1 to φ10 mm, and the wavelength of the laser beam can be set to 800 to 10,000 nm. As the laser beam, a fiber laser (a wavelength of 1070 nm), a YAG laser (a wavelength of 1064 nm), a laser diode (808 nm, 840 nm, 940 nm), or a laser that generates a wavelength as described later can be used.

Furthermore, in a case where heating is performed by laser emission while the pressurization is applied, and particularly a case where the shaft inner layer contains the laser beam-absorbing material, the shaft inner layer generates heat by a laser beam, and then reaches the melting point, resulting in an increase in fluidity. As a result, the shaft inner layer is easily pushed out toward the distal member side. It is considered that this series of configurations causes a shape in which the distal portion of the shaft inner layer intrudes in an arc outward in the radial direction from the inner surface of the distal member. Moreover, in the case where heating is performed by laser emission while the pressurization is applied, and particularly the case where the shaft inner layer contains the laser beam-absorbing material, the shaft outer layer is under the most pressure, and the distal end of the shaft is further tapered by heating, so that the inner layer and the outer layer are likely to be integrated.

The pressurization on the elastic body is finally released, and a bonded body with the distal member and the shaft fused can be taken out.

The balloon catheter can be manufactured by adopting the same steps (A) to (C) as described above through the bonded body and the balloon core material.

In the balloon catheter according to the suitable embodiment of the present invention, as apparent from the measurement results of the crystallinity distribution described later, the members do not mix with each other to form a lump even though the interface between the members has a layer that is slightly melted and mixed in melting of the members. It is considered that in a case where a lump is interposed between the members, cracks may be generated due to a difference in mechanical properties between the base material of the members and the lump. In the suitable embodiment of the present invention, the shapes of the members themselves in the cross-sectional view in the axial direction are changed as compared between before and after molding, but the members maintain the positional relationship in the radial direction. The same applies to a member previously formed with a plurality of layers by co-extrusion molding, such as the catheter shaft or the balloon, and all the layers do not mix with each other to form a lump, except that the outer layer and the inner layer are mixed at the balloon distal portion. In the comparison between before and after molding, the shapes of the layers themselves in the cross-sectional view in the axial direction are changed, but the members maintain the positional relationship in the radial direction. The reason for this is not necessarily clear, but it is presumed that since radiation heating is performed with a laser having an optimum wavelength when laser emission is performed at a predetermined output while the pressurization is applied, the layers are melted while being maintained with minimum necessary heating. That is, in a case of heating with a heat-shrinkable tube, the difference in the degree of melting between the materials in the thickness direction increases due to heat transfer from the heat-shrinkable tube, and the excessive flowing occurs. On the other hand, in the case of heating by laser beam emission, it is considered that the difference in the degree of melting between the members in the thickness direction is small, so that the melting is achieved, with less flowing.

## Example

The effects of the present invention will be described with reference to the following Examples and Comparative Examples. Note that the technical scope of the present invention is not limited only to the following Examples. In the following examples, unless otherwise specified, operations were performed at room temperature (25°C). Furthermore, unless otherwise specified, "%" and "parts" mean "% by weight" and "parts by weight", respectively.

### <Preparation of Catheter with Distal Tip and Balloon Catheter>

### 1. Material

#### 1-1. Material for Distal Tip

A polyamide elastomer (PEBAX7033SA01MED, manufactured by Arkema Inc.) and a polyamide elastomer (VESTAMID E40-S1, manufactured by Polyplastics-Evonik Corporation) were mixed at a ratio of 8 : 2 (weight ratio) to

obtain a mixture. A pigment (DAIREN BLUE NPN-4970, manufactured by DIC Corporation, phthalocyanine blue: 33.30% by weight, carbon black: 1.72% by weight) was blended in the mixture so that the mixing amount was 0.7% by weight to prepare a material for a distal tip.

1-2. Material for Shaft Inner Layer

Carbon nanotubes (MWNT-7, manufactured by Hodogaya Chemical Co., Ltd.) were blended in a polyamide (Grilamid L16, manufactured by EMS-CHEMIE AG) so that the blending amount was 5% by weight to prepare a material for a shaft inner layer.

1-3. Material for Shaft Outer Layer

A polyamide elastomer (Grilflex ELG6260, manufactured by EMS-CHEMIE AG) and a polyamide (Rilsamid AESNO TL, manufactured by Arkema Inc.) were mixed at a ratio of 3 : 7 (weight ratio) to prepare a material for a shaft outer layer.

1-4. Material for Balloon Inner Layer, Balloon Outer Layer, and Balloon Middle Layer

As a material for an inner layer and an outer layer of the balloon, a polyamide elastomer (Grilflex ELG5660, manufactured by EMS-CHEMIE AG) was used. As a material for a balloon middle layer (base layer), a polyamide (Grilamid L25, manufactured by EMS-CHEMIE AG) was used. All the layers were transparent members containing no laser beam-absorbing material.

2. Component Molding

2-1. Distal Tip

The material for the distal tip was extruded while being heated to form a tube for the distal tip.

2-2. Shaft

A tube for the shaft was formed by co-extrusion of the material for the shaft inner layer and the material for the shaft outer layer.

2-3. Balloon

The material for the balloon inner layer, the material for the balloon middle layer, and the material for the balloon outer layer were molded into a tube by co-extrusion while being heated, and then molded into a balloon shape by biaxial stretching.

3. Fusion Apparatus

3-1. Laser Processing Machine

A laser irradiation unit emits a laser beam having a wavelength that causes the fusion portion to generate heat by radiation heating. The spot diameter of the laser beam can be set to φ0.1 to φ10 mm, and the wavelength of the laser beam can be set to 800 to 10,000 nm. The wavelength of the laser beam was appropriately selected from these ranges according to the workpiece. In a case of fusion between the members in which the laser beam-absorbing material is blended, the wavelength of the laser beam can be selected from 800 to 5,000 nm, preferably 900 to 2,300 nm. In a case of fusion between the transparent members or between the member in which the laser beam-absorbing material is blended and the transparent member, the wavelength of the laser beam can be selected from 1,300 to 2,500 nm, preferably 1,500 to 2,300 nm. The laser beam is substantially emitted in a direction orthogonal to the axial direction of the workpiece. The laser beam may be emitted at an angle appropriately changed according to the workpiece.

3-2. Pressurizing Apparatus

A hollow elastic body presses the workpiece by applying an external force to the hollow elastic body made of silicone. The hollow elastic body made of silicone has laser transmission properties. The term "laser transmission

properties" means that the workpiece contains a material having a transmittance of 80% or more with respect to a laser beam per 1 mm of the thickness of the workpiece in the radial direction. The workpiece passes through a metal core material and is pressed by the hollow elastic body in the axial direction of the core material. The workpiece, the core material, the hollow elastic body, and a support thereof rotate about the axis of the core material.

4. Manufacturing Method

4-1. Catheter with Distal Tip

The tube for the distal tip and the tube for the shaft were inserted through the core material and passed into the hollow elastic body in a state where both tubes were abutted to each other. The inner diameter of the hollow elastic body is larger than the outer diameter of the workpiece, and the workpiece and the hollow elastic body are not in contact with each other before pressing. Both tubes (workpiece) were rotated about the axis of the core material, an external force was applied to the hollow elastic body, and a laser beam was emitted, so that a radially inward force was applied to the workpiece while the members including the portions to which the laser beam was emitted were heated. In particular, in the distal tip and the inner layer of the shaft with the laser beam-absorbing material blended, the temperatures of these members increased before the temperatures of the other members increased. After a lapse of a predetermined time, laser beam emission and pressurization by the hollow elastic body were stopped. Once the heat dissipated, the workpiece was removed from the core material. In this way, a catheter with a distal tip (inner diameter: 0.41 mm, an outer diameter: 0.58 mm) was produced.

4-2. Balloon Catheter

The catheter with a distal tip manufactured in 4-1 described above was inserted through the core material, and a predetermined fusion position of the distal end of the balloon was arranged to span the boundary portion between the distal tip and the shaft. These were passed into the elastic body so that the hollow elastic body covered the predetermined fusion position. The inner diameter of the hollow elastic body is larger than the outer diameter of the workpiece, and the workpiece and the hollow elastic body are not in contact with each other before pressing. The workpiece was rotated about the axis of the core material, pressurization was applied to the elastic body from the outside, and a laser beam was emitted, so that a radially inward force was applied to the workpiece while the members including the portions to which the laser beam was emitted were heated. In particular, in the distal tip and the inner layer of the shaft with the laser beam-absorbing material blended, the temperatures of these members increased before the temperatures of the other members increased. After a lapse of a predetermined time, laser beam emission and pressurization by the hollow elastic body were stopped. Once the heat dissipated, the workpiece was removed from the core material. In this way, a balloon catheter (inner diameter: 0.41 mm, an outer diameter: 0.57 mm) was produced.

<Evaluation of Crystallinity>

1. Selection of Crystalline Band and Amorphous Band

1-1. Conditions

Apparatus name: FTS7000e manufactured by Agilent Technologies, Inc./Infrared microscope UMA600
Measurement method: Microscopic-transmission method
Integration: 128 times
Resolution: 4 $cm^{-1}$
Temperature conditions: The temperature was increased from room temperature to 230°C, IR measurements were performed, a sample was then slowly cooled to room temperature at a rate sufficient for crystallization to occur, followed by IR measurement. The IR measurement was performed in nitrogen.

Pretreatment: A sample was embedded in an epoxy resin and processed with a microtome into a thin section having a thickness of about 10 μm. Resins 2 and 3 were processed into 10 μm thin sections with a microtome. Resins 1, 4, and 5 were processed into thin sections with a cutter.
The resins 1 to 5 were as follows.
Resin 1: Tube made of material for distal tip
Resin 2: Polyamide elastomer pellets (Grilflex ELG5660, manufactured by EMS-CHEMIE AG: material for balloon inner layer and balloon outer layer)
Resin 3: Polyamide pellets (Grilamid L25, manufactured by EMS-CHEMIE AG: material for balloon middle layer)
Resin 4: Tube made of material for shaft outer layer
Resin 5: Tube made of material for shaft inner layer

The tube was molded by extruding each resin while heating.

1-2. Method

Absorbance with respect to a wavelength was measured as follows using the resins 1 to 5, and a crystalline band and an amorphous band were selected.

· Resin 1 (distal tip)

1) The resin 1 (tubular) was processed into a 10 μm thin section with a cutter.
2) 13 peaks (C1 to C13) were confirmed (upper part of Fig. 7) as crystalline bands (bands that disappear upon melting) from the IR spectrum (Fig. 7) obtained by heating the above-described thin section to 230°C and then cooling and observed before and after melting. As the bands to be used for the evaluation of crystallinity, C9 that is a peak with minimal overlap and baseline drift was selected.
3) As amorphous bands (bands remaining even in a molten state), five peaks (A1 to A5) were confirmed (lower part of Fig. 7). As the bands to be used for the evaluation of crystallinity, A3 that is a peak with minimal overlap and baseline drift was selected.
4) In the evaluation of the crystallinity, the peak intensity ratio of the crystalline band C9 and the amorphous band A3 was used as an index.

· Resin 2 (balloon inner layer and balloon outer layer)

1) The resin 2 (pellet-like) was processed into a 10 μm thin section with a microtome.
2) 11 peaks (C1 to C11) were confirmed (upper part of Fig. 8) as crystalline bands (bands that disappear upon melting) from the IR spectrum (Fig. 8) obtained by heating the above-described thin section to 230°C and then cooling and observed before and after melting. As the bands to be used for the evaluation of crystallinity, C7 that is a peak with minimal overlap and baseline drift was selected.
3) As amorphous bands (bands remaining even in a molten state), five peaks (A1 to A5) were confirmed (lower part of Fig. 8). As the bands to be used for the evaluation of crystallinity, A3 that is a peak with minimal overlap and baseline drift was selected.
4) In the evaluation of the crystallinity, the peak intensity ratio of the crystalline band C7 and the amorphous band A3 was used as an index.

· Resin 3 (balloon middle layer)

1) The resin 3 (pellet-like) was processed into a 10 μm thin section with a microtome.
2) 13 peaks (C1 to C13) were confirmed (upper part of Fig. 9) as crystalline bands (bands that disappear upon melting) from the IR spectrum (Fig. 9) obtained by heating the above-described thin section to 230°C and then cooling and observed before and after melting. As the bands to be used for the evaluation of crystallinity, C7 that is a peak with minimal overlap and baseline drift was selected.
3) As amorphous bands (bands remaining even in a molten state), seven peaks (A1 to A7) were confirmed (lower part of Fig. 9). As the bands to be used for the evaluation of crystallinity, A3 that is a peak with minimal overlap and baseline drift was selected.
4) In the evaluation of the crystallinity, the peak intensity ratio of the crystalline band C7 and the amorphous band A3 was used as an index.

· Resin 4 (shaft outer layer)

1) The resin 4 (tubular) was processed into a 10 μm thin section with a cutter.
2) 13 peaks (C1 to C13) were confirmed (upper part of Fig. 10) as crystalline bands (bands that disappear upon melting) from the IR spectrum (Fig. 10) obtained by heating the above-described thin section to 230°C and then cooling and observed before and after melting. As the bands to be used for the evaluation of crystallinity, C7 that is a peak with minimal overlap and baseline drift was selected.
3) As amorphous bands (bands remaining even in a molten state), six peaks (A1 to A6) were confirmed (lower part of Fig. 10). As the bands to be used for the evaluation of crystallinity, A3 that is a peak with minimal overlap and baseline drift was selected.
4) In the evaluation of the crystallinity, the peak intensity ratio of the crystalline band C7 and the amorphous band A3 was used as an index.

· Resin 5 (shaft inner layer)

1) The resin 5 (tubular) was processed into a 10 μm thin section with a cutter.
2) 13 peaks (C1 to C13) were confirmed (upper part of Fig. 11) as crystalline bands (bands that disappear upon melting) from the IR spectrum (Fig. 11) obtained by heating the above-described thin section to 230°C and then cooling and observed before and after melting. As the bands to be used for the evaluation of crystallinity, C7 that is a peak with minimal overlap and baseline drift was selected.
3) As amorphous bands (bands remaining even in a molten state), seven peaks (A1 to A7) were confirmed (lower part of Fig. 11). As the bands to be used for the evaluation of crystallinity, A3 that is a peak with minimal overlap and baseline drift was selected.
4) In the evaluation of the crystallinity, the peak intensity ratio of the crystalline band C7 and the amorphous band A3 was used as an index.

In the above description, each resin is collected from pellets or members before product processing, but a band to be used may be selected by directly collecting a thin section of a resin from the product.

2. Measurement of Crystallinity Distribution by Imaging IR

2-1. Conditions

Apparatus name: Cary600 manufactured by Agilent Technologies, Inc./Infrared microscope Cary670
Measurement method: Microscopic-transmission method
Measurement range: 700 μm square (128 pixel square)
Element size: 5.5 μm square
Integration: 128 times
Spectral resolution: 4 $cm^{-1}$
Pretreatment: A sample was embedded in an epoxy resin and processed with a microtome into a thin section having a thickness of about 10 μm.

2-2. Method

1) A balloon catheter (Example) prepared as described above and a balloon catheter (Comparative Example) prepared using the same material as that in Example by the method described in JP 2001-191412 A were prepared as samples.
2) A thin section was prepared with the above-described pretreatment so that the measurement range was from the distal tip to the part where the balloon and shaft were fused.
3) The crystalline band and the amorphous band specified in 1-2 described above were measured for each pixel, and

in order to reduce the effect of variations in the sample thickness, a ratio was calculated as the "crystallinity" of the sample, with the amorphous band as the denominator and the crystalline band as the numerator (Equation 1). A color image was used to clarify the crystallinity distribution of the sample. The crystalline band and the amorphous band of each resin specified in 1-2 described above were selected as follows. A crystalline band of 1161 $cm^{-1}$ common to each resin was selected. The width of the amorphous band was observed to range from 1366 to 1359 $cm^{-1}$ during melting (lower part in Figs. 7 to 11), but the width after cooling (upper part in Figs. 7 to 11) was located around 1369 $cm^{-1}$, so that the amorphous band was defined at 1369 $cm^{-1}$.

In this example, the crystalline band and the amorphous band were common due to the resins of the same series, but in a case where different bands are selected for different resins, each band is selected by the following method to create an image of the crystallinity distribution. That is, first, when a crystalline band and an amorphous band are selected by the method in 1-2 described above, a resin having an absorption peak that does not overlap with other resins is specified, and the crystalline band and the amorphous band of the resin are selected. Next, a region of the resin specified in the imaging IR is designated, and a crystallinity is calculated from an equation in which the crystalline band and amorphous band in Equation 2 are replaced with those selected for different resins on the basis of data of the crystalline band and the amorphous band in the designated region, and a color image corresponding to a numerical value of the crystallinity is incorporated into a color image of another resin.
[Math. 2]

$$\text{Crystallinity} = \frac{\text{Intensity of crystalline band (1161 cm}^{-1}\text{)}}{\text{Intensity of amorphous band (1369 cm}^{-1}\text{)}} \times 100\ [\%] \qquad \text{(Equation 2)}$$

3. Results and Discussion

Fig. 12 illustrates the measurement results of the crystallinity distribution in the axial direction of the balloon catheter by the imaging IR. The measurement results in Fig. 12 and the subsequent figures illustrate one radial side of the distal portion of the balloon catheter. In Fig. 12, the balloon distal end (left side) and the shaft distal end (right side) in the balloon catheter of Comparative Example are indicated by arrows. In Fig. 12, the lower left is the distal side of the balloon catheter, and the upper right is the proximal side of the balloon catheter. In Fig. 12, the crystallinity is expressed by color, and is continuously transferred to red (about 60%), orange, yellow, green, light blue, blue, and purple (0%).

As illustrated in Fig. 12, it can be seen that the balloon catheter of Example has lower crystallinity than that of Comparative Example. Furthermore, it can be seen that the balloon catheter of Example is different from the balloon catheter of Comparative example in that the crystallinity on the distal side of the balloon middle layer is lower than the crystallinity on the proximal side. Therefore, it can be seen that the balloon catheter of Example has an improved flexibility as compared with the balloon catheter of Comparative Example.

Fig. 13 illustrates relationships between measurement results of crystallinity distribution confirmed by the imaging IR in the balloon catheter.

As illustrated in Fig. 13(C), the distal portion of the balloon middle layer includes a wedge portion that is wedge-shaped, and the wedge portion has a parallel portion parallel to the axial direction. Accordingly, it is considered that the physical properties gradually shift from the distal side to the proximal side of the balloon middle layer, and the fusion portion is thus smoothly bent.

As illustrated in Fig. 13(A), the crystallinity of the balloon outer layer is lower than the crystallinity of the balloon inner layer. In the shaft inner layer and the balloon middle layer, the crystallinity decreases from the proximal side toward the distal side, and particularly in the balloon middle layer, the crystallinity gradually decreases from the proximal side toward the distal side. More specifically, in the balloon middle layer, the crystallinity on the distal side (mostly green) is about 1.5% to 6% lower than the crystallinity on the proximal side (mostly orange) (crystallinity is 35.7%, 37.5%, 40.0%, 41.2% from the distal side). As a result, it is considered that since the hardness gradually changes from the distal side to the proximal side, it is possible to form gradient physical properties, contribute to the improvement of passability in a living body, and maintain the flexibility against bending in the vicinity of the fusion portion.

As described Fig. 13(A), the thickness of the distal side of the balloon middle layer is larger than the thickness of the shaft at the portion bonded to the balloon, and is equal to or slightly smaller than the thickness (not illustrated) of the shaft at the site closer to the proximal side than the portion where the balloon is bonded to the shaft. Accordingly, it is considered that the thickness of the balloon middle layer is incorporated at the portion where the balloon is bonded to the shaft, and an increase in rigidity can be suppressed.

As can be seen from Fig. 13(B), the crystallinity of each of the proximal side of the distal tip and the distal region of the balloon distal portion is less than 40% (green to light blue). Accordingly, it is considered that the strength can be improved while maintaining the flexibility of the fusion portion. Furthermore, it is considered that since the difference in

crystallinity from the adjacent resin is small, the occurrence of cracks such as delamination can be reduced.

In particular, there is a large area exhibiting a crystallinity of less than 20% (blue) in the distal region of the balloon distal portion. Although the crystallinity of the distal end of the shaft is higher than that of the distal region of the balloon distal portion, it is considered that gradient physical properties can be achieved by cushioning the hardness of the shaft with the distal region of the balloon distal portion.

The crystallinity (the average value of crystallinity of the distal tip in the region defined by a perpendicular line drawn with respect to the axial direction at a position of 200 μm on the distal side away from the most distal end of the shaft inner layer) of the proximal side of the distal tip was 28%.

In the distal region of the balloon distal portion (the region defined by a perpendicular line drawn with respect to the axial direction at a position of 150 μm on the proximal side away from the most distal end of the balloon), a region exhibiting a crystallinity of less than 20% was 42%. The crystallinity of the distal region of the balloon distal portion was 21.5%.

<Evaluation of Flexibility>

1. Cantilever Bending Test

1-1. Conditions

Speed: 5 mm/min
Distance between clamping jig and plate: 0.5 mm
Stroke: 0.3 mm
Measurement portion: distal tip (plate is pressed toward part A with part B sandwiched by jig)
Fusion margin (plate is pressed toward part B with part C sandwiched by jig)

Fig. 14 illustrates relationships between measurement positions and IR images. The definitions of parts A, B, and C are as follows.

Part B: Interface between distal tip (blue) and shaft (black)
Part A: 0.5 mm on the distal side away from part B
Part C: 0.5 mm on the proximal side away from part B

1-2. Correction for Outer Diameter Difference (Ratio of Second Moment of Area)

Distal tip: Point B (0.0054/0.0039)
Fusion margin: Point C (0.0159/0.0116)

[Table 1]

| | Part A | Part B | Part C |
|---|---|---|---|
| Comparative Example | 0.55 mm | 0.61 mm | 0.77 mm |
| Second moment of area | 0.0034 $mm^4$ | 0.0054 $mm^4$ | 0.0159 $mm^4$ |
| Example | 0.52 mm | 0.57 mm | 0.72 mm |
| Second moment of area | 0.0021 $mm^4$ | 0.0039 $mm^4$ | 0.0116 $mm^4$ |

1-3. Results and Discussion

The results of the cantilever bending test are illustrated in Fig. 15.

As illustrated in Fig. 13(B), in Example, the crystallinity of each of the distal side of the shaft inner layer and the proximal side of the distal tip is less than 40% (green to light blue), the crystallinity on the distal side of the balloon middle layer is around 40% (green), and the crystallinity of the balloon middle layer is lower on the distal side than on the proximal

side. On the other hand, as illustrated in Fig. 12, in Comparative Example, the crystallinity of the distal end of the shaft is more than 40% and is around 50% (orange), and the crystallinity of the balloon distal end is around 50% (orange) in the axial direction.

In the balloon catheter of Example, the crystallinities on the distal side of the shaft inner layer, the proximal side of the distal tip, and the distal side of the balloon middle layer is lower than those of Comparative Example. As a result, as illustrated in Fig. 15, from the test results of the flexibility of the distal tip, it can be seen that the result of Example (40.0 gf after outer diameter correction) under the load pushed by 0.3 mm with the cantilever is about 23% more flexible than the result of Comparative Example (51.8 gf). Furthermore, from the flexibility test results regarding the balloon distal portion and the fusion portion (fusion margin) of the shaft, it can be seen that the result of Example (37.0 gf after outer diameter correction) is about 30% more flexible than the result of Comparative Example (52.8 gf).

<Preparation of Catheter 2 with Distal Tip and Balloon Catheter 2>

1. Material

1-1. Material 2 for Distal Tip

A pigment (DAIREN ORANGE PPD-3865, manufactured by DIC Corporation, containing 8.57% by weight of Perylene Red and 8.57% by weight of condensed azo-yellow) was blended in a polyamide elastomer (Griflex ELG6260, manufactured by EMS-CHEMIE AG) so that the blending amount was 5% by weight to obtain a mixture. The mixture and a polyamide elastomer (Griflex ELG5660, manufactured by EMS-CHEMIE AG) were mixed at a ratio of 7 : 3 (weight ratio) to prepare a material 2 for a distal tip.

1-2. Material for Shaft Inner Layer

A pigment (DAIREN Black PPB-0491, manufactured by DIC Corporation, containing 25% by weight of carbon black) was blended in a polyamide (DIAMID (registered trademark) L1940W, manufactured by Polyplastics-Evonik Corporation) so that the blending amount was 20% by weight to prepare a material 2 for a shaft inner layer.

1-3. Material for Shaft Outer Layer

A polyamide (DIAMID (registered trademark) L1940W, manufactured by Polyplastics-Evonik Corporation) and a polyamide elastomer (Grilflex ELG6260, manufactured by EMS-CHEMIE AG) were mixed at a ratio of 8 : 2 (weight ratio) to prepare a material 2 for a shaft outer layer.

1-4. Material for Balloon Inner Layer, Balloon Outer Layer, and Balloon Middle Layer

As a material 2 for a balloon inner layer and a material 2 for a balloon outer layer, a polyamide elastomer (Grilflex ELG6260, manufactured by EMS-CHEMIE AG) was used. As a material 2 for a balloon middle layer (base layer), a polyamide (Grilamid L25, manufactured by EMS-CHEMIE AG) was used. All the layers were transparent members containing no laser beam-absorbing material.

2. Component Molding

2-1. Distal Tip

The material 2 for the distal tip was extruded while being heated to form a tube 2 for the distal tip.

2-2. Shaft

A tube 2 for the shaft was formed by co-extrusion of the material for the shaft inner layer and the material for the shaft outer layer.

2-3. Balloon

The material for the balloon inner layer, the material for the balloon middle layer, and the material for the balloon outer layer were molded into a tube by co-extrusion while being heated, and then molded into a balloon 2 by biaxial stretching.

3. Fusion Apparatus

As a laser processing machine and a pressurizing apparatus, the fusion apparatus similar to the preparation of the catheter with a distal tip and the balloon catheter described above was used.

4. Manufacturing Method

The tube 2 for the distal tip and the tube 2 for the shaft were inserted through a core material and passed into a hollow elastic body in a state where both tubes were abutted to each other. The inner diameter of the hollow elastic body is larger than the outer diameter of the workpiece, and the workpiece and the hollow elastic body are not in contact with each other before pressing. Both tubes (workpiece) were rotated about the axis of the core material, an external force was applied to the hollow elastic body, and a laser beam was emitted, so that a radially inward force was applied to the workpiece while the members including the portions to which the laser beam was emitted were heated. In particular, in the distal tip and the inner layer of the shaft with the laser beam-absorbing material blended, the temperatures of these members increased before the temperatures of the other members increased. After a lapse of a predetermined time, laser beam emission and pressurization by the hollow elastic body were stopped. Once the heat dissipated, the workpiece was removed from the core material. In this way, a catheter 2 with a distal tip (inner diameter: 0.41 mm, an outer diameter: 0.58 mm) was produced.

4-2. Balloon Catheter 2

The catheter 2 with a distal tip manufactured in 4-1 described above was inserted through the core material, and a predetermined fusion position of the distal end of the balloon 2 was arranged to span the boundary portion between the distal tip and the shaft. These were passed into the elastic body so that the hollow elastic body covered the predetermined fusion position. The inner diameter of the hollow elastic body is larger than the outer diameter of the workpiece, and the workpiece and the hollow elastic body are not in contact with each other before pressing. The workpiece was rotated about the axis of the core material, pressurization was applied to the elastic body from the outside, and a laser beam was emitted, so that a radially inward force was applied to the workpiece while the members including the portions to which the laser beam was emitted were heated. In particular, in the distal tip and the inner layer of the shaft with the laser beam-absorbing material blended, the temperatures of these members increased before the temperatures of the other members increased. After a lapse of a predetermined time, laser beam emission and pressurization by the hollow elastic body were stopped. Once the heat dissipated, the workpiece was removed from the core material. In this way, a balloon catheter 2 (inner diameter: 0.41 mm, an outer diameter: 0.57 mm) was produced.

<Evaluation of Crystallinity>

The crystallinity of the balloon catheter 2 was evaluated in the same manner as in the above-described balloon catheter.

The balloon catheter 2 of Example had a structure as illustrated in Fig. 5. The distal portion of the shaft (or the shaft inner layer) intrudes in an arc outward in the radial direction from the inner surface of the distal tip. Accordingly, it is considered that the contact area with the adjacent resin is increased, and necessary and sufficient bonding strength can be obtained even with a short fusion length. It is considered that a flexible distal end can be obtained as the fusion length is short.

In the balloon middle layer, the crystallinity (43.2%) on the distal side of the balloon distal portion was lower than the crystallinity (45.0%) on the proximal side of the balloon distal portion. Furthermore, in the balloon middle layer, the crystallinity on the distal side of the balloon distal portion was about 1.8% lower than the crystallinity on the proximal side of the balloon distal portion. As a result, it is considered that since the hardness gradually changes from the distal side to the proximal side, it is possible to form gradient physical properties, contribute to the improvement of passability in a living body, and maintain the flexibility against bending in the vicinity of the fusion portion.

The present application is based on Japanese Application No. 2022-156478 filed on September 29, 2022, the entire content of which is incorporated herein by reference.

Reference Signs List

3 Balloon catheter
3A Distal portion of balloon catheter

10, 110 Shaft
130 Elastic body
20, 320 Distal member
150 Core material
320H Inner surface of distal member
60 Balloon
60A Distal portion of balloon
60A1 Wedge portion
60A2 Parallel portion
60D Distal end of balloon
65 Proximal portion of balloon
66 Inner layer of balloon
67 Base layer of balloon
68 Outer layer of balloon
69 Mixed layer
270 X-ray radiopaque marker
280 Guide wire
310 Shaft
310H Lumen of shaft
311 Outer tube shaft
311A Distal portion of outer tube shaft
311H Lumen of outer tube shaft
312 Inner tube shaft
312A Distal portion of inner tube shaft
312R Proximal opening of inner tube shaft
313 Outer layer of inner tube shaft
313A Distal portion of outer layer of inner tube shaft
314 Inner layer of inner tube shaft
314A Distal portion of inner layer of inner tube shaft
315 Guide wire lumen
340 Hub
341 Port of hub

## Claims

**1.** A balloon catheter comprising:

a shaft that extends in an axial direction; and
a balloon that is disposed on an outer periphery of the shaft, wherein
in a distal portion of the balloon, a crystallinity on a distal side is lower than a crystallinity on a proximal side.

**2.** The balloon catheter according to claim 1, wherein the crystallinity of the distal portion of the balloon gradually decreases from the proximal side toward the distal side.

**3.** The balloon catheter according to claim 1 or 2, wherein the crystallinity on the distal side of the distal portion of the balloon is 1.5% to 6% lower than the crystallinity on the proximal side.

**4.** The balloon catheter according to claim 1 or 2, wherein in a cross-sectional view in the axial direction,

the distal portion of the balloon includes
a wedge portion that is wedge-shaped, and
the wedge portion includes a parallel portion parallel to the axial direction.

**5.** The balloon catheter according to claim 4, wherein

the balloon includes an inner layer, a base layer, and an outer layer, and
in the parallel portion, a crystallinity of the outer layer is lower than a crystallinity of the inner layer.

**6.** The balloon catheter according to claim 5, wherein the inner layer and the outer layer contain an elastomer.

**7.** The balloon catheter according to claim 1 or 2, further comprising a distal member that is fused to a distal side of the shaft and is more flexible than the shaft, wherein a crystallinity of each of a proximal portion of the distal member and a distal region of the distal portion of the balloon is less than 40%.

**8.** The balloon catheter according to claim 1 or 2, wherein a thickness at a distal end of the balloon positioned at a portion fused to the shaft is equal to or smaller than a thickness of the shaft at a site closer to the proximal side than a portion where the balloon is bonded to the shaft, and is larger than a thickness of the shaft at a portion bonded to the balloon.

FIG. 1

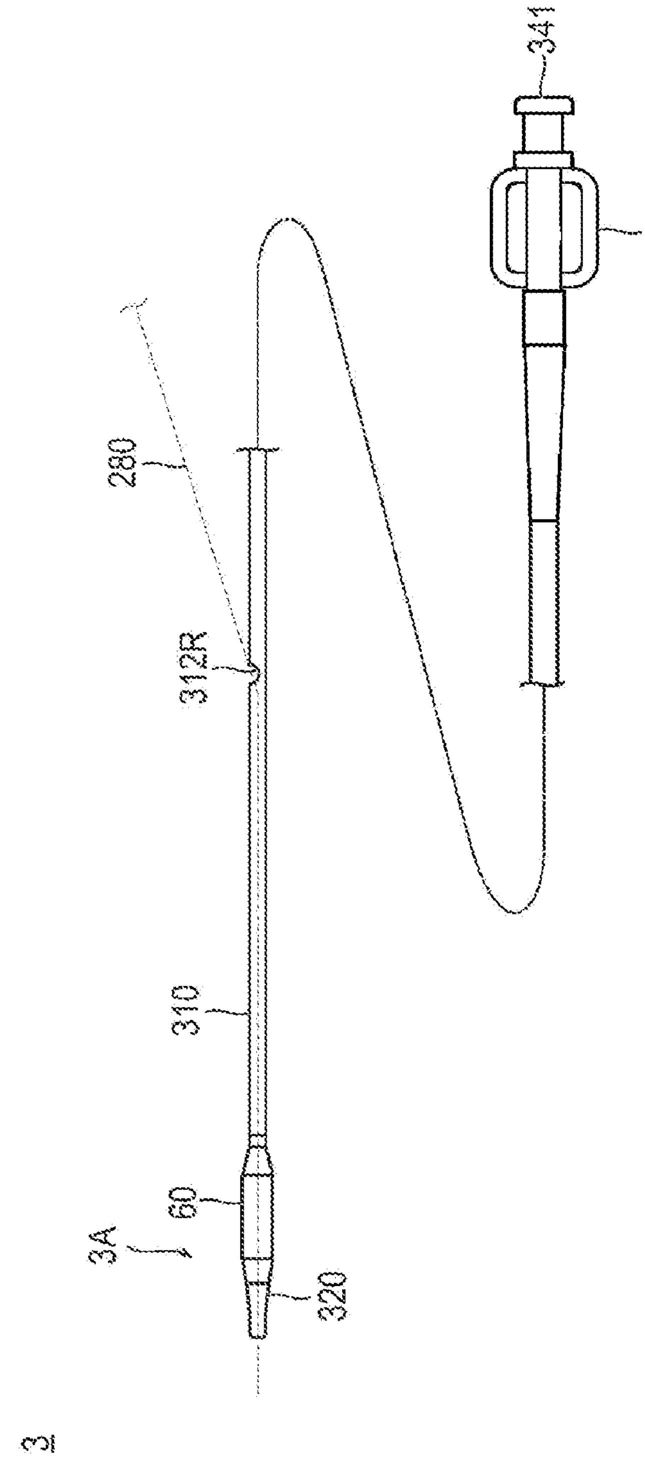

FIG. 2

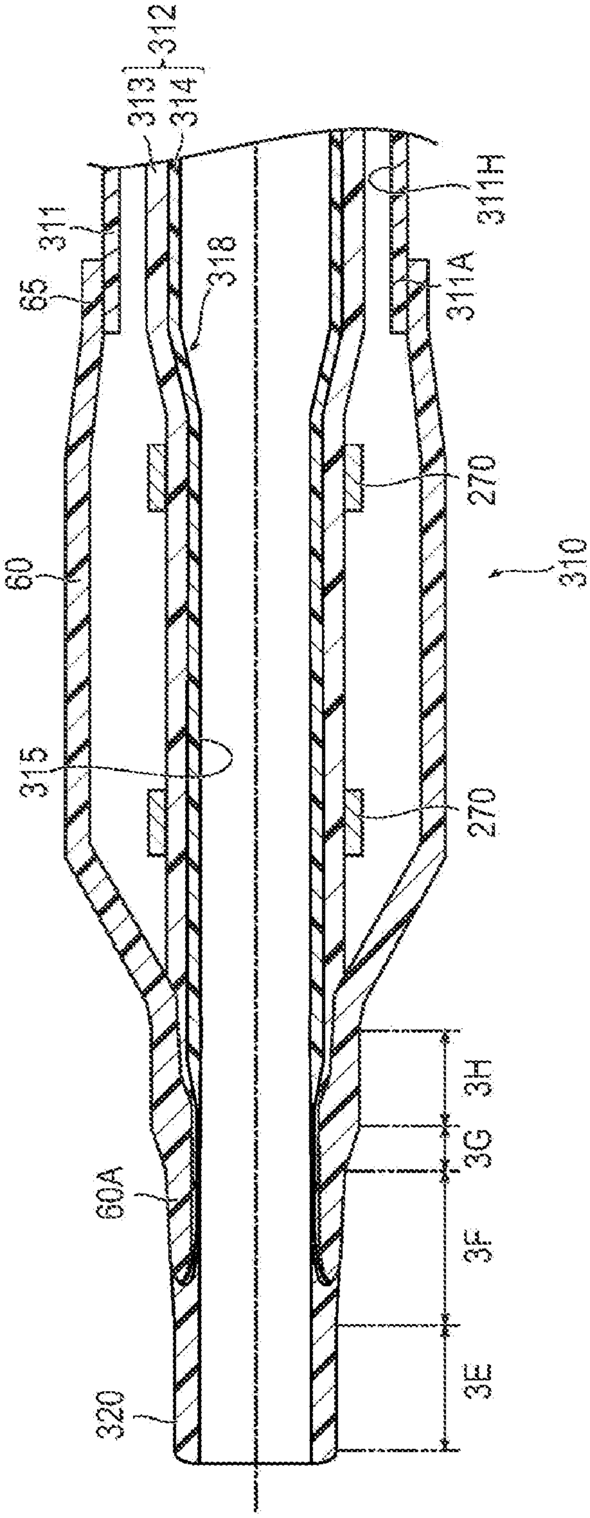
312
313
314
311
65
318
311H
311A
270
60
310
315
270
60A
3H
3G
3F
320
3E

FIG. 3A

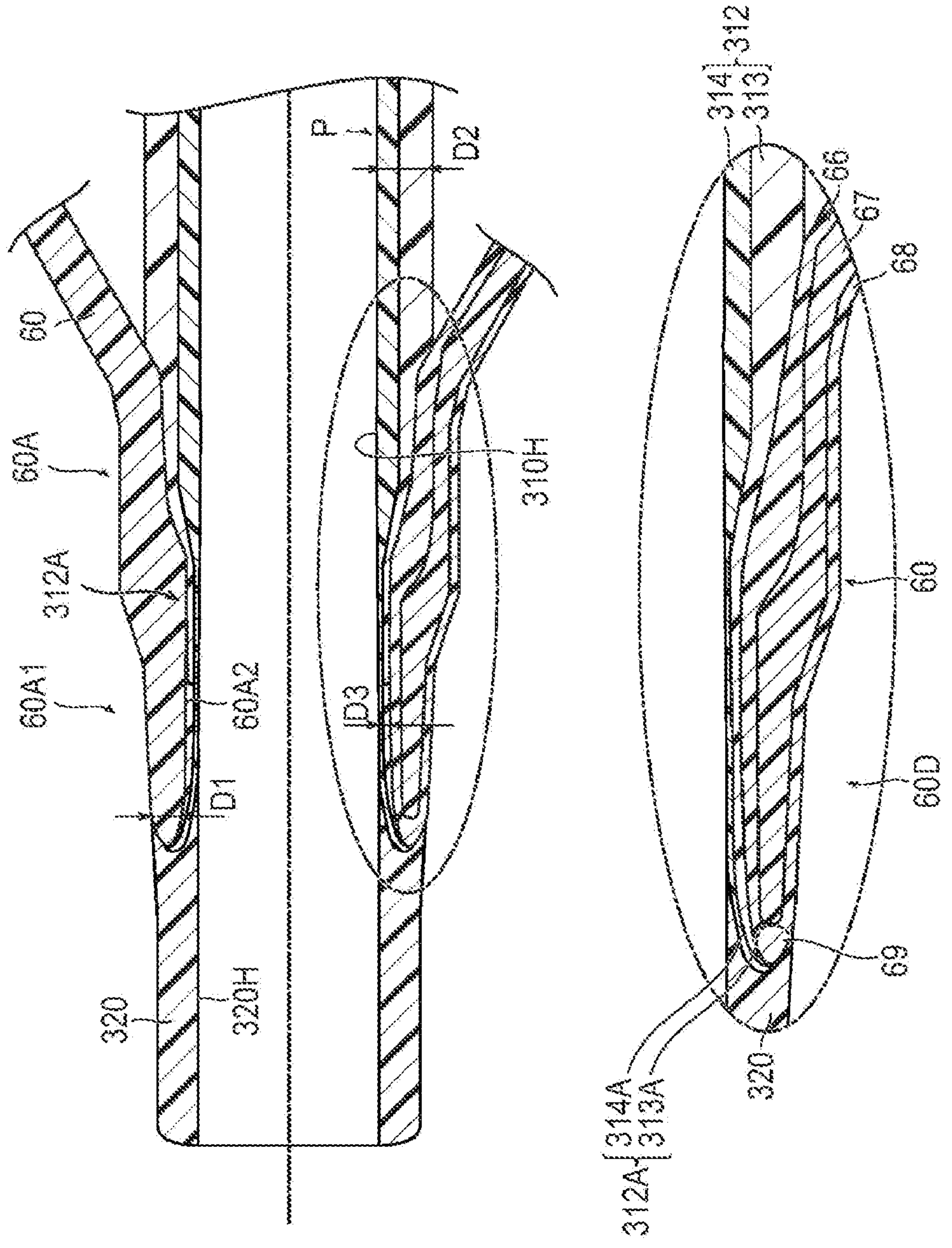
60
60A
312A
60A1
60A2
D1
320
320H
P
D2
310H
D3
314
313
312
66
67
68
60
60D
69
320
312A
314A
313A

FIG. 3B

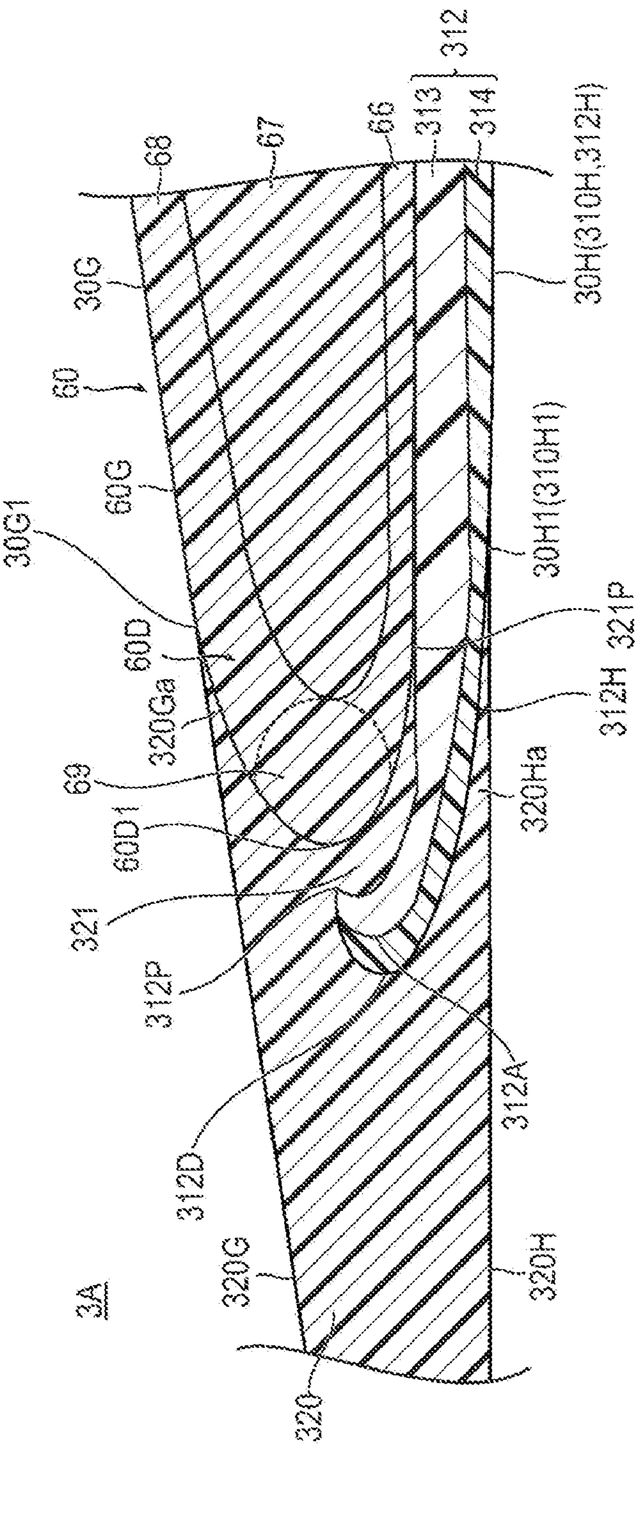

312
313
314
68
67
66
30G
60
60G
30G1
60D
320Ga
69
60D1
321
312P
312D
320G
3A
320
30H(310H,312H)
30H1(310H1)
321P
312H
320Ha
312A
320H

FIG. 4

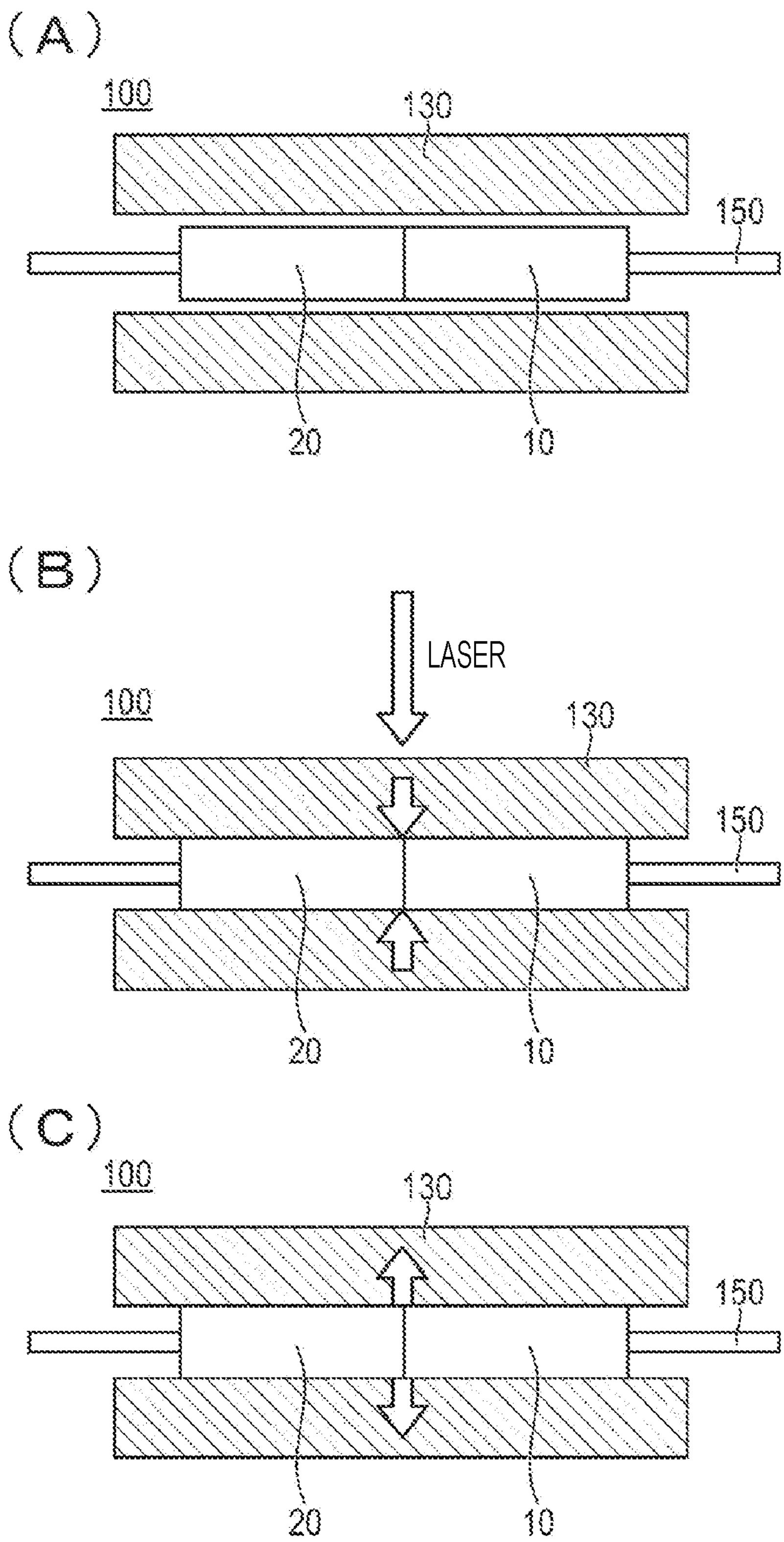

*FIG. 5*

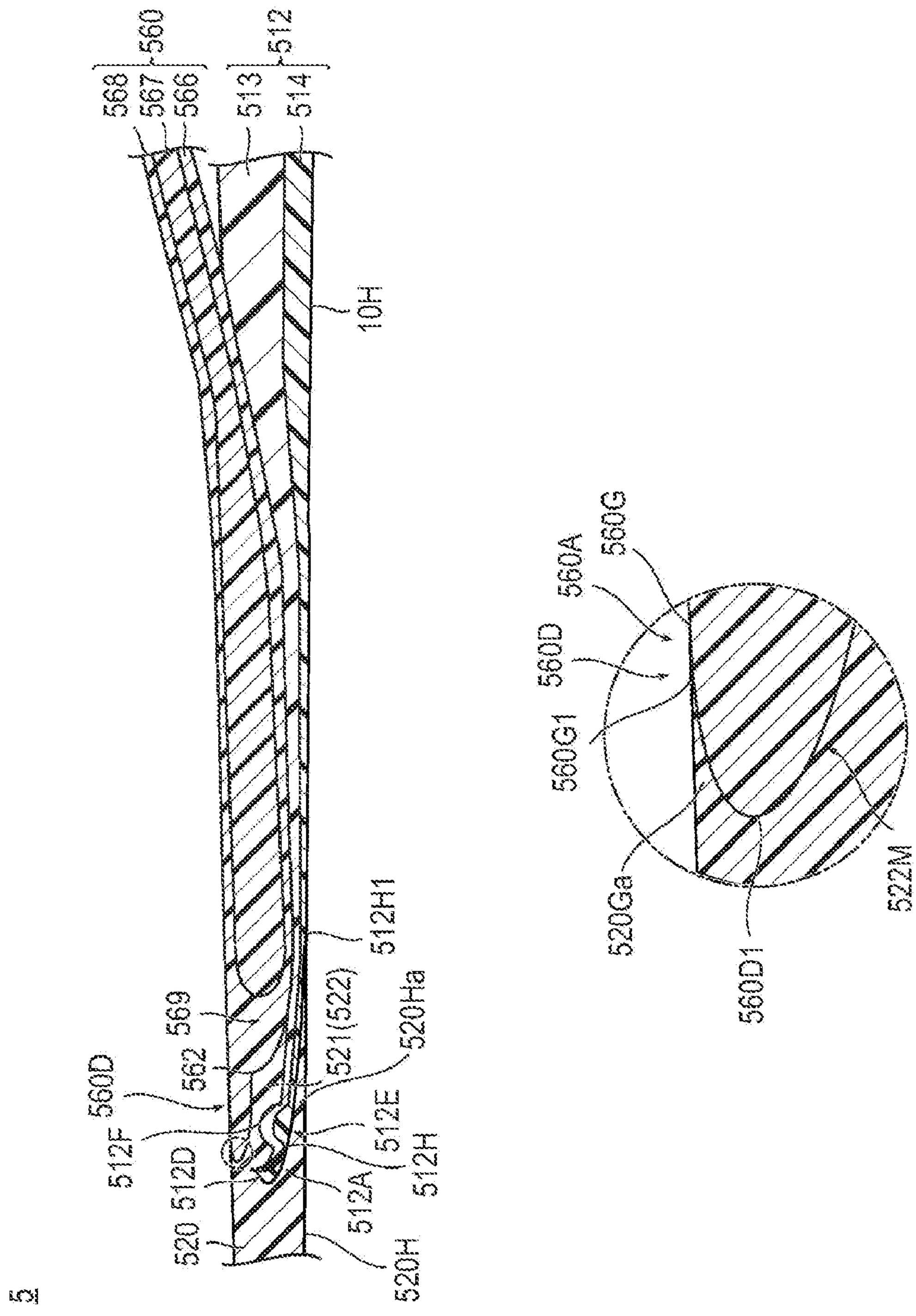
5
560
568
567
566
512
513
514
10H
512H1
521(522)
520Ha
569
562
560D
512F
512D
520
512E
512H
512A
520H
560G
560A
560D
560G1
520Ga
560D1
522M

FIG. 6

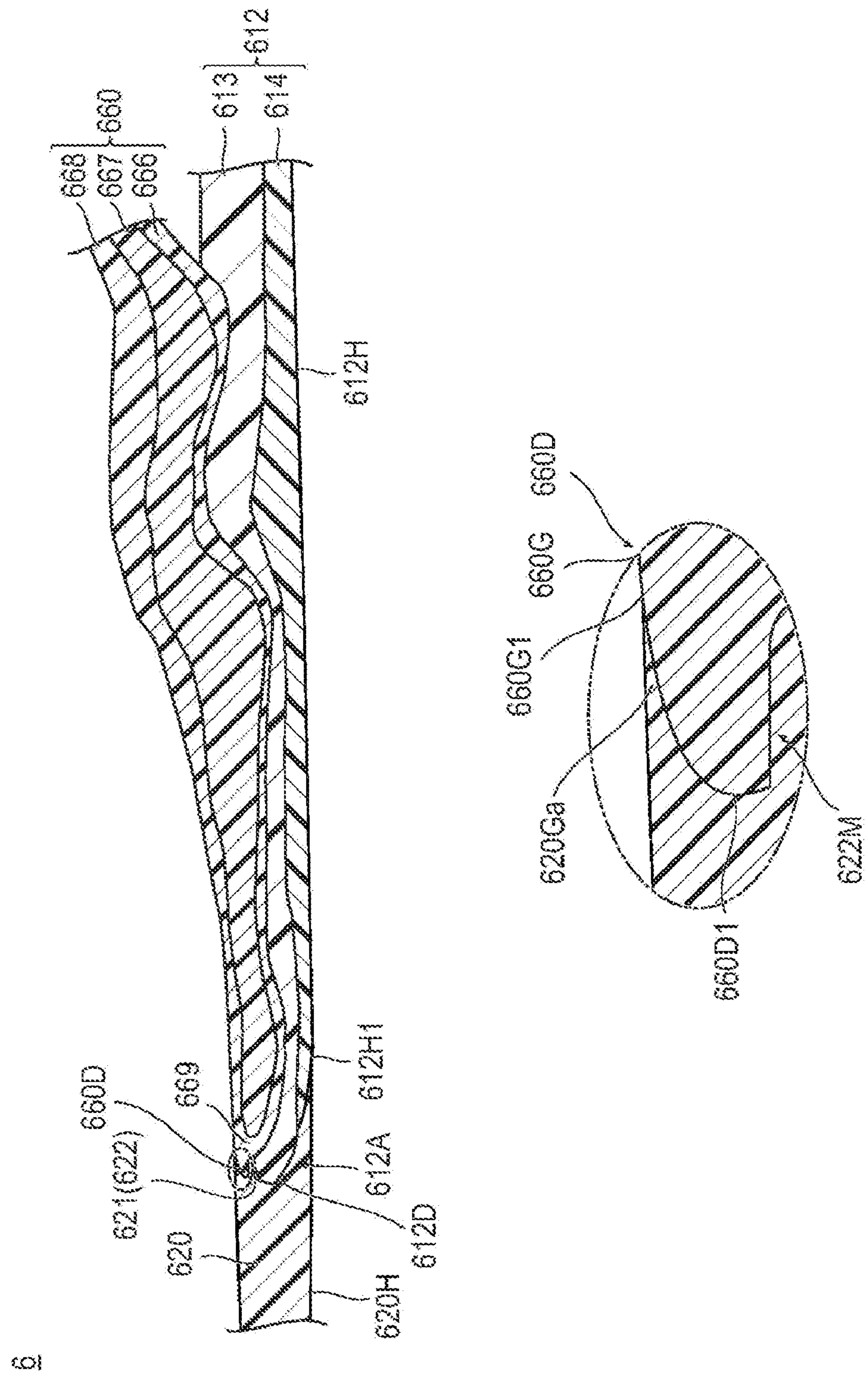
6
612
613
614
660
666
667
668
612H
660D
660G
660G1
620Ga
622M
660D1
669
612H1
612A
612D
621(622)
620
620H

FIG. 7

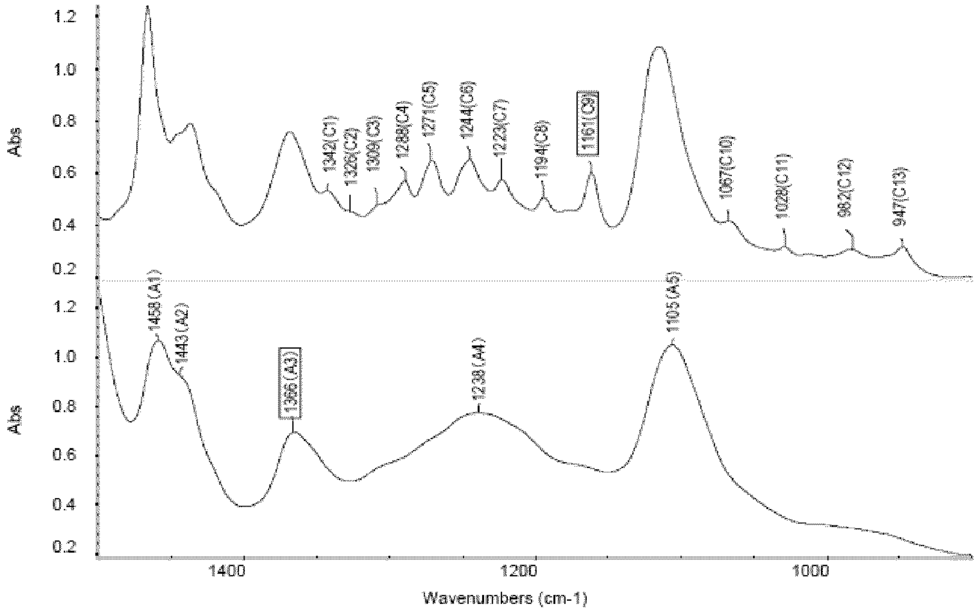
Abs
1342(C1)
1326(C2)
1309(C3)
1288(C4)
1271(C5)
1244(C6)
1223(C7)
1194(C8)
1161(C9)
1067(C10)
1028(C11)
982(C12)
947(C13)
1458 (A1)
1443 (A2)
1366 (A3)
1238 (A4)
1105 (A5)
1400
1200
1000
Wavenumbers (cm-1)

FIG. 8

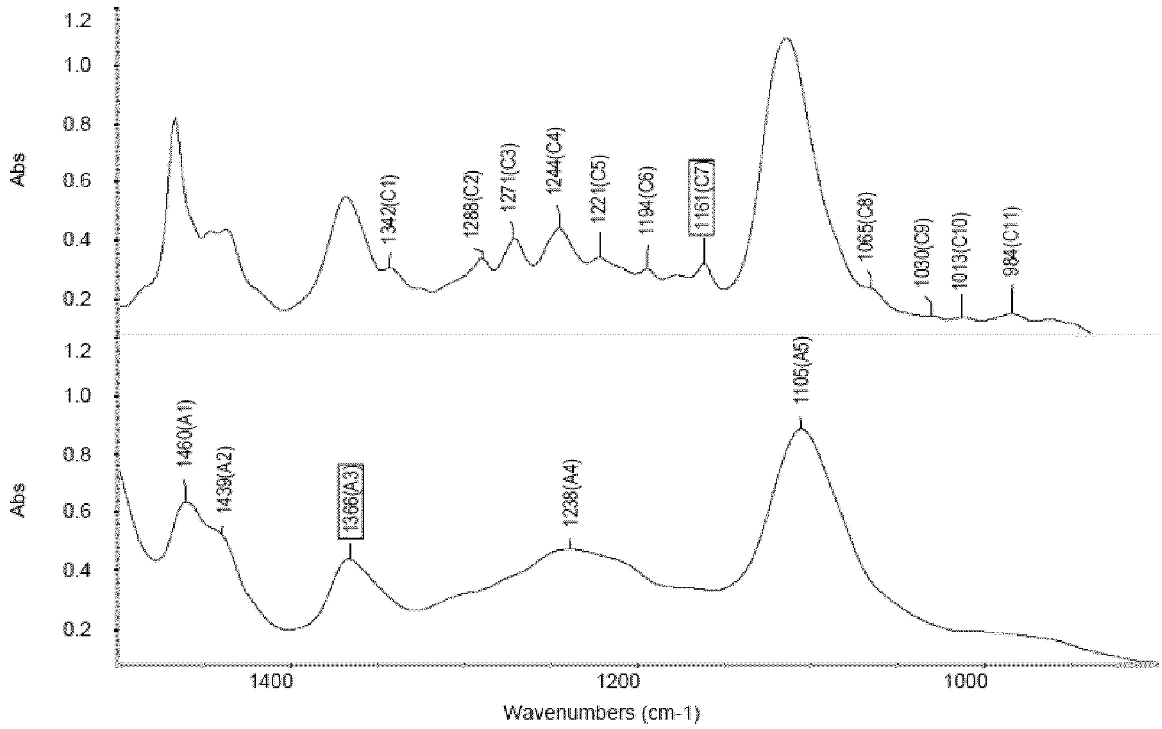
Abs
1342(C1)
1288(C2)
1271(C3)
1244(C4)
1221(C5)
1194(C6)
1161(C7
1065(C8)
1030(C9)
1013(C10)
984(C11)
1460(A1)
1439(A2)
1366(A3)
1238(A4)
1105(A5)
1400
1200
1000
Wavenumbers (cm-1)

FIG. 9

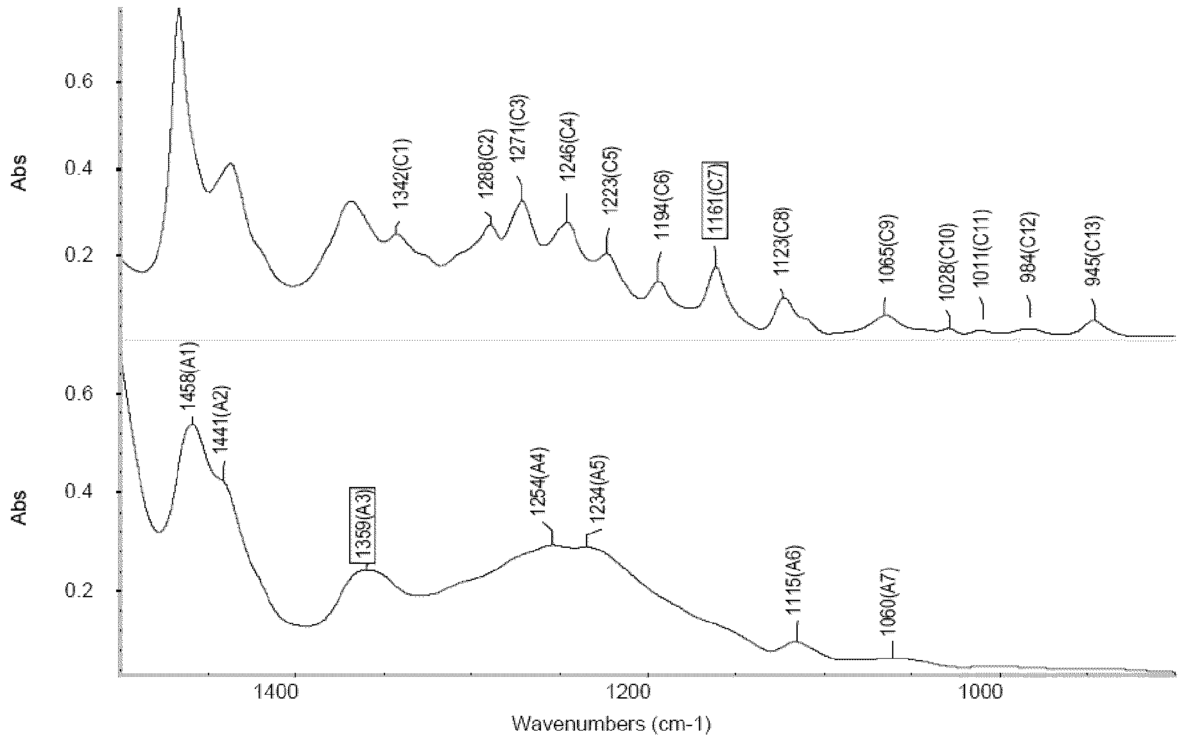
0.6
0.4
0.2
Abs
1342(C1)
1288(C2)
1271(C3)
1246(C4)
1223(C5)
1194(C6)
1161(C7)
1123(C8)
1065(C9)
1028(C10)
1011(C11)
984(C12)
945(C13)
1458(A1)
1441(A2)
1359(A3)
1254(A4)
1234(A5)
1115(A6)
1060(A7)
1400
1200
1000
Wavenumbers (cm-1)

FIG. 10

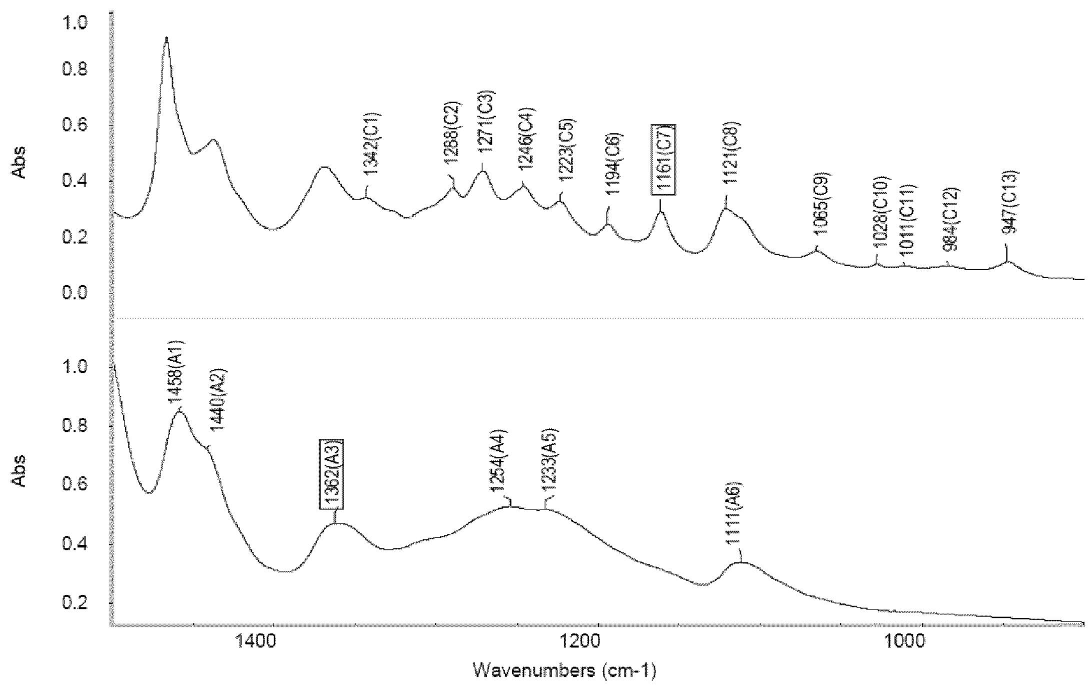
1.0
0.8
0.6
0.4
0.2
0.0
Abs
1342(C1)
1288(C2)
1271(C3)
1246(C4)
1223(C5)
1194(C6)
1161(C7)
1121(C8)
1065(C9)
1028(C10)
1011(C11)
984(C12)
947(C13)
1458(A1)
1440(A2)
1362(A3)
1254(A4)
1233(A5)
1111(A6)
1400
1200
1000
Wavenumbers (cm-1)

FIG. 11

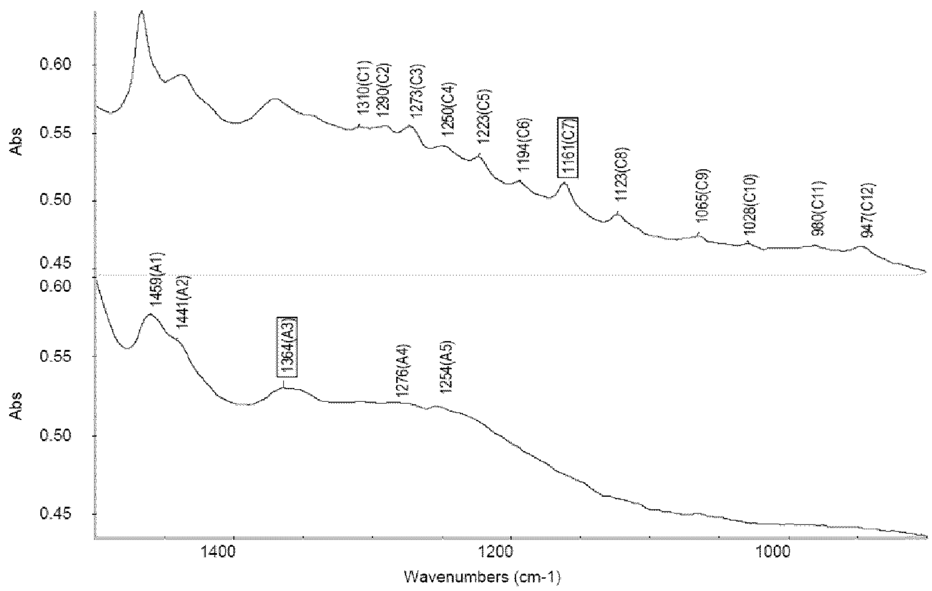
Abs
0.60
0.55
0.50
0.45
1310(C1)
1290(C2)
1273(C3)
1250(C4)
1223(C5)
1194(C6)
1161(C7)
1123(C8)
1065(C9)
1028(C10)
980(C11)
947(C12)
1459(A1)
1441(A2)
1364(A3)
1276(A4)
1254(A5)
1400
1200
1000
Wavenumbers (cm-1)

FIG. 12

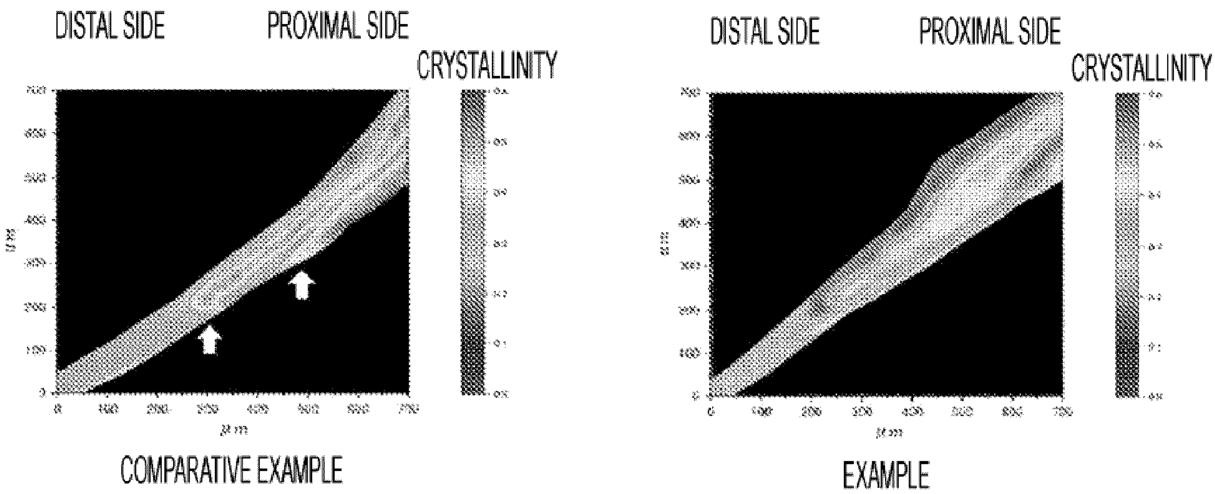
DISTAL SIDE
PROXIMAL SIDE
CRYSTALLINITY
COMPARATIVE EXAMPLE
DISTAL SIDE
PROXIMAL SIDE
CRYSTALLINITY
EXAMPLE

FIG. 13

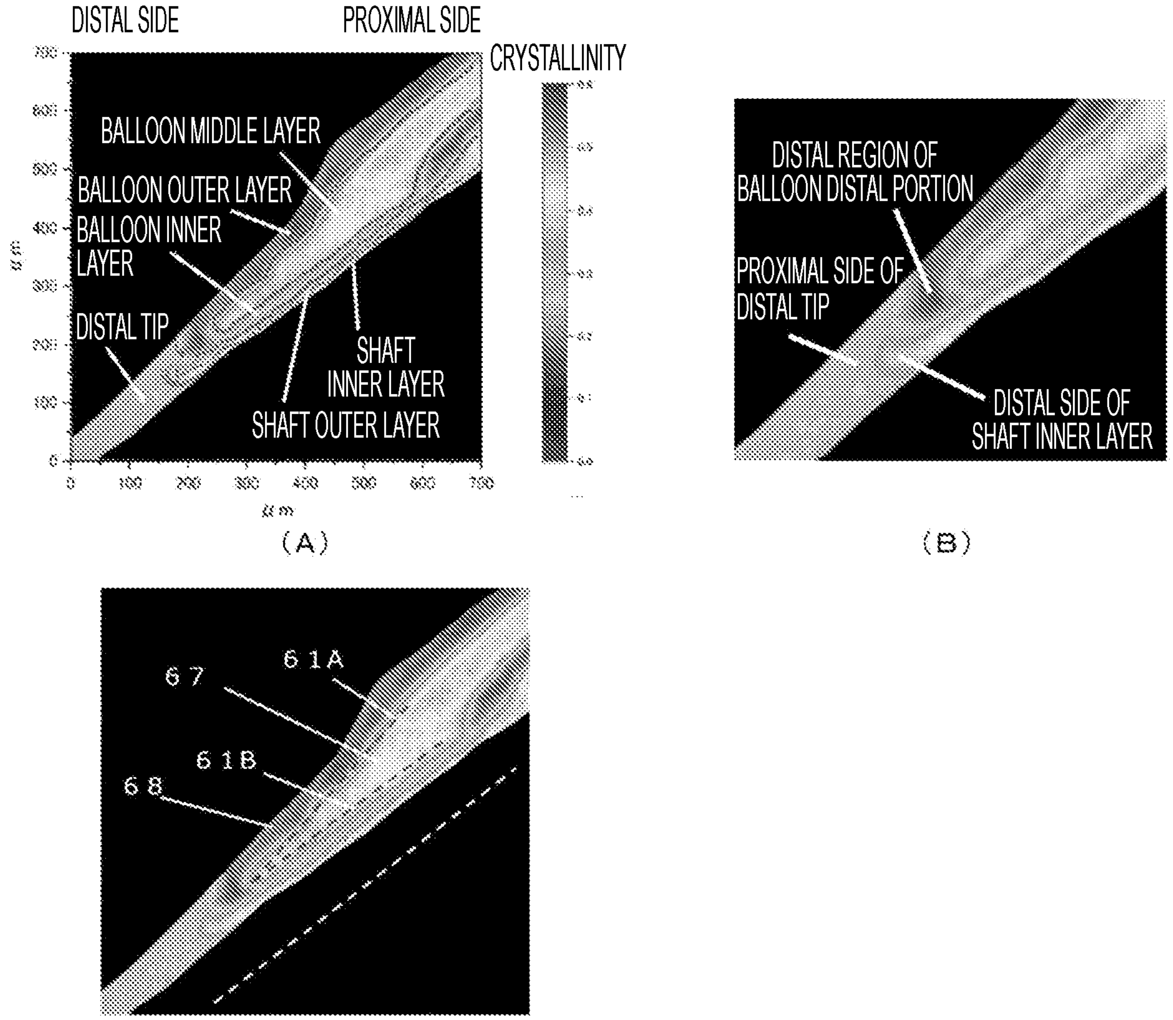


(C)

FIG. 14

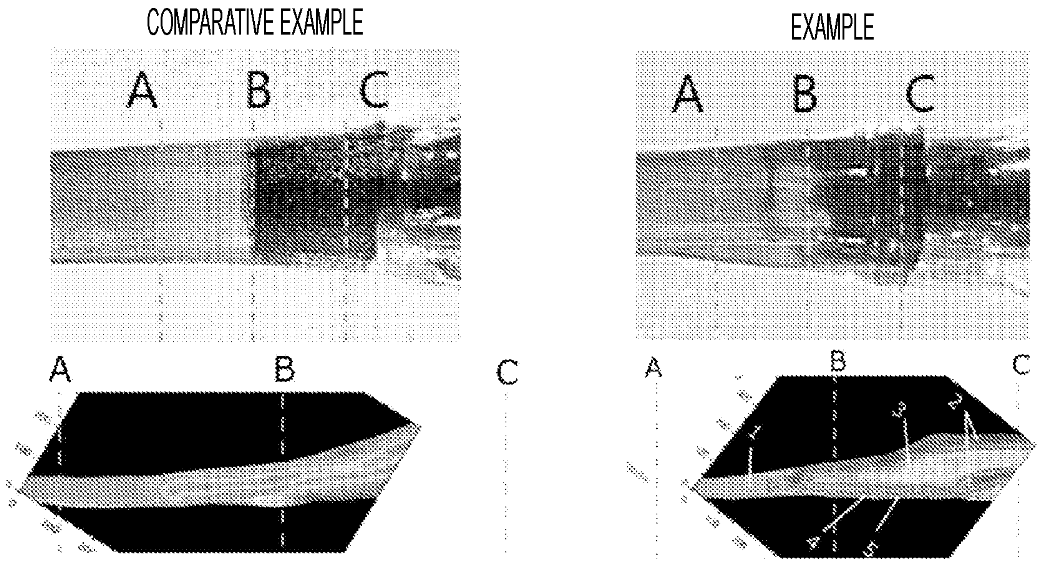

FIG. 15

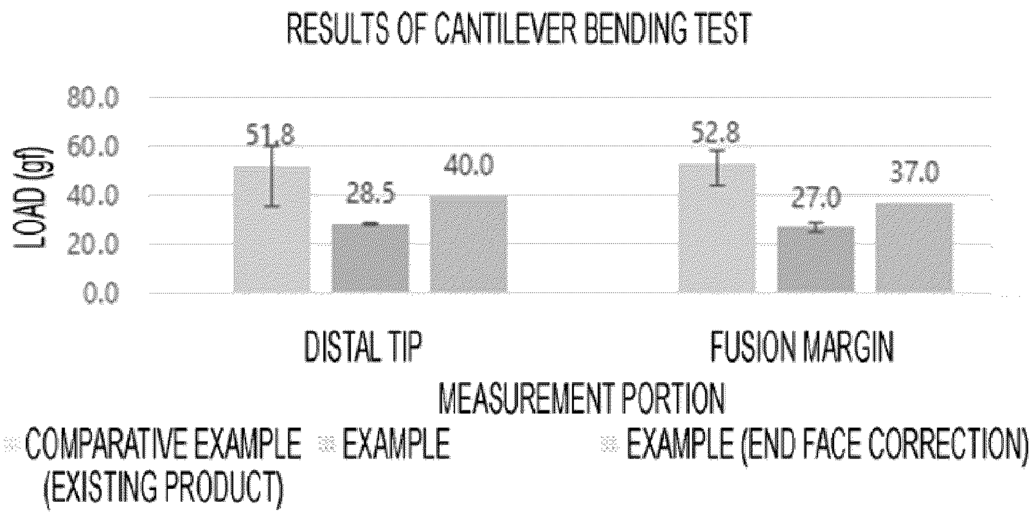
RESULTS OF CANTILEVER BENDING TEST
80.0
60.0
40.0
20.0
0.0
LOAD (gf)
51.8
28.5
40.0
52.8
27.0
37.0
DISTAL TIP
FUSION MARGIN
MEASUREMENT PORTION
COMPARATIVE EXAMPLE (EXISTING PRODUCT)
EXAMPLE
EXAMPLE (END FACE CORRECTION)

## INTERNATIONAL SEARCH REPORT

International application No.
**PCT/JP2023/034940**

**A. CLASSIFICATION OF SUBJECT MATTER**

***A61M 25/10***(2013.01)i
FI: A61M25/10

According to International Patent Classification (IPC) or to both national classification and IPC

**B. FIELDS SEARCHED**

Minimum documentation searched (classification system followed by classification symbols)

A61M25/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2022-20124 A (ASAHI INTECC CO., LTD.) 01 February 2022 (2022-02-01) paragraphs [0024]-[0029], fig. 1 | 1-3 |
| Y | paragraphs [0024]-[0029], fig. 1 | 4-7 |
| A | paragraphs [0024]-[0029], fig. 1 | 8 |
| Y | JP 2017-055859 A (ASAHI INTECC CO., LTD.) 23 March 2017 (2017-03-23) fig. 5 | 4-6 |
| Y | JP 2005-538744 A (BOSTON SCIENTIFIC LTD.) 22 December 2005 (2005-12-22) paragraph [0022], fig. 3 | 4-6 |
| Y | JP 10-024098 A (TERUMO CORP.) 27 January 1998 (1998-01-27) paragraphs [0044]-[0060], fig. 2 | 5-6 |
| Y | JP 2020-039376 A (TERUMO CORP.) 19 March 2020 (2020-03-19) paragraphs [0024], [0026], fig. 3 | 7 |
| A | WO 2013/111700 A1 (TERUMO CORP.) 01 August 2013 (2013-08-01) paragraphs [0041]-[0044], [0062] | 1-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2023** | **28 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/034940**

**C. DOCUMENTS CONSIDERED TO BE RELEVANT**

| Category* | Citation of document, with indication, where appropriate, of the relevant passages | Relevant to claim No. |
|---|---|---|
| A | JP 2012-143381 A (GOODMAN CO., LTD.) 02 August 2012 (2012-08-02) entire text, all drawings | 1-8 |

Form PCT/ISA/210 (second sheet) (January 2015)

**INTERNATIONAL SEARCH REPORT**
**Information on patent family members**

International application No.
**PCT/JP2023/034940**

| Patent document cited in search report | Publication date (day/month/year) | Patent family member(s) | Publication date (day/month/year) |
|---|---|---|---|
| JP 2022-20124 A | 01 February 2022 | (Family: none) | |
| JP 2017-055859 A | 23 March 2017 | US 2017/0071613 A1<br>fig. 5<br>US 2018/0325533 A1<br>EP 3144028 A1<br>CN 106540368 A<br>KR 10-2017-0032825 A<br>CN 110478602 A | |
| JP 2005-538744 A | 22 December 2005 | US 2003/0078613 A1<br>paragraph [0028], fig. 3<br>WO 2003/035158 A2<br>CA 2462604 A | |
| JP 10-024098 A | 27 January 1998 | (Family: none) | |
| JP 2020-039376 A | 19 March 2020 | WO 2018/135533 A1<br>paragraphs [0024], [0026], fig. 3 | |
| WO 2013/111700 A1 | 01 August 2013 | US 2014/0309533 A1<br>paragraphs [0053]-[0056], [0075]<br>US 2019/0275290 A1<br>EP 2808051 A1<br>CN 104053471 A | |
| JP 2012-143381 A | 02 August 2012 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H05192410 A
- JP 2005160536 A
- US 4100309 A
- JP S5919582 A
- JP 2001191412 A
- JP 2022156478 A